# EUROPEAN PATENT APPLICATION

(11) **EP 3 888 653 A1**
(43) Date of publication of application: **06.10.2021**
(21) Application number: 19888537.8
(22) Date of filing: 29.11.2019
(51) Int. Cl.: A61K 31/444, A61P 7/02, A61P 9/00, C07D 471/04

(54) **NOVEL CRYSTAL OF (3S)-3-[2-(6-AMINO-2-FLUOROPYRIDINE-3-YL)-4-FLUORO-1H-IMIDAZOLE-5-YL]-7-[5-CHLORO-2-(1H-TETRAZOLE-1-YL)PHENYL]-2,3-DIHYDROINDOLIZINE-5(1H)-ONE**

(30) Priority: 30.11.2018 JP 2018224434
(71) Applicant: ONO Pharmaceutical Co., Ltd., Osaka-shi, Osaka 541-8526 (JP)
(72) Inventor: FUJITO, Takayuki, Mishima-gun, Osaka 618-8585 (JP); ONO, Shizuka, Mishima-gun, Osaka 618-8585 (JP); OHTANI, Shuhei, Mishima-gun, Osaka 618-8585 (JP)
(74) Representative: J A Kemp LLP
(86) International application number: PCT/JP2019/046893
(87) International publication number: WO 2020/111268

(57) **Abstract**

Provided is a novel crystal form of (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one.

Provided is (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-3-hydroxybenzoic acid (1/1).

## Description

### TECHNICAL FIELD

The present invention relates to a novel crystal of (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one and others.

### BACKGROUND ART

Thrombosis and thromboembolism which is a complication of thrombosis (hereinafter referred to as thromboembolic disease) are ranked high along with cancer as the cause of death of adults, and have become important problems in recent years. Thromboembolic disease occurs by the formation of a thrombus at a site of vascular injury. Alternatively, thromboembolic disease occurs when a thrombus is released and is carried by the blood stream into another blood vessel where the thrombus obstructs a blood vessel at another site. Thromboembolic disease includes, for example, venous thromboembolism which is a collective term for deep venous thrombosis and pulmonary embolism, cerebral stroke, angina pectoris, myocardial infarction, other various arterial and venous thrombosis and the like.

Tissue factor expressed on a vascular wall due to the injury of a blood vessel and the like becomes the starting point of the blood coagulation cascade and forms a complex with blood coagulation factor VII which is present in blood in a very small quantity. This complex activates blood coagulation factor IX and blood coagulation factor X, and activated blood coagulation factor X converts prothrombin to thrombin. Thrombin converts fibrinogen to fibrin and finally insoluble fibrin is formed (the initial stage). It is supposed that thrombin produced in the process promotes the formation of a thrombus at the initial stage and is important for hemostasis. On the other hand, it has been reported that thrombin activates blood coagulation factor XI and causes explosive thrombin production via activated blood coagulation factor XI (hereinafter also referred to as FXIa) (the amplification stage), which results in an increase in thrombi (see Non Patent Literatures 1 to 3).

For the treatment and/or prevention of thromboembolic disease, anticoagulant agents are generally used. Though conventional anticoagulant agents exhibit excellent antithrombotic actions, bleeding complications, which are serious side effects, have been problematic. Alternatively, in order not to cause bleeding complications, the doses of the agents are limited and it is supposed that there is a possibility that the agents do not exhibit sufficient antithrombotic actions. Under such conditions, an agent for treating and/or preventing thrombosis and thromboembolism having a novel mechanism of action, which suppresses the growth of or increase in pathological thrombi and does not affect the formation of hemostatic thrombi, is required. As one of the targets of the agent, FXIa is attracting attention in recent years. Blood coagulation factor XI is one of plasma serine proteases which are involved in the regulation of blood coagulation and becomes FXIa by activated blood coagulation factor XII, thrombin or itself. FXIa is one of constituents of the blood coagulation pathway which is referred to as the intrinsic system or the contact system in the classical blood coagulation cascade and activates blood coagulation factor IX by selectively cleaving peptide bonds of Arg-Ala and Arg-Val. The safety of FXIa is supported by the observations that the blood coagulation factor XI deficiency in humans, which is called hemophilia C, results in mild to moderate bleeding characterized primarily by postoperative or posttraumatic hemorrhage. In addition, the effects and the high safety of FXIa are demonstrated by the experimental results of experimental thrombosis and bleeding models which used blood coagulation factor XI deficient mice and the experimental results of an anti-blood coagulation factor XI neutralizing antibody or an antisense in experimental thrombosis and bleeding models which used monkeys or rabbits, in addition to the results of observations of the blood coagulation factor XI deficiency in humans (see Non Patent Literatures 4 to 8).

Based on the above results, it is expected that FXIa is a very attractive target without exhibiting the side effect of bleeding when developing an antithrombotic agent for treatment and/or prevention and an FXIa inhibitor becomes a very potent and safe antithrombotic agent for treatment or prevention without having any undesirable side effects such as bleeding.

In Patent Literature 1, it is described that (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one is a selective blood coagulation factor XIa inhibitor and is therefore useful as a therapeutic agent for thromboembolic diseases and the like.

### CITATIONS LISTS

### Patent Literature

Patent Literature 1: International Publication No. 2016/093285 pamphlet Non Patent Literatures

Non Patent Literature 1: Blood Coagulation and Fibrinolysis, 2006, Vol. 17, pages 251-257
Non Patent Literature 2: Science, 1991, Vol. 253, pages 909-912
Non Patent Literature 3: Blood, 2003, Vol. 102, pages 953-955
Non Patent Literature 4: Journal of Thrombosis and Haemostasis, 2005, Vol. 3, pages 695-702
Non Patent Literature 5: Journal of Thrombosis and Haemostasis, 2006, Vol. 4, pages 1982-1988
Non Patent Literature 6: Blood, 2012, Vol. 119, pages 2401-2408
Non Patent Literature 7: Blood, 2009, Vol. 113, pages 936-944
Non Patent Literature 8: Journal of Thrombosis and Haemostasis, 2006, Vol. 4, pages 1496-1501

### SUMMARY OF INVENTION

### TECHNICAL PROBLEMS

As an active ingredient for a pharmaceutical, a crystal which is easy to handle and is superior in hygroscopicity and oral absorption is desirable. The physical properties of (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one disclosed in Patent Literature 1 are examined, and it is found that the crystallinity of a free form and a dihydrochloride of this compound are extremely poor. Furthermore, it is also found that, although the dihydrate can have a crystal form, the dihydrochloride shows high hygroscopicity and low oral absorption as mentioned below. In these situations, it has been demanded to produce a novel active pharmaceutical ingredient form (e.g., a salt, a solvate, a cocrystal) which can form a crystal thereof and is superior in handleability when used in a pharmaceutical. Accordingly, the object of the present invention is to form a crystal of a salt, a solvate or a cocrystal of (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one and provide an active pharmaceutical ingredient form which is superior in low hygroscopicity and/or oral absorption.

### SOLUTIONS TO PROBLEMS

In order to solve the above-mentioned problems, the present inventors have made studies. As a result, it is found that (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one can form a crystal in conjunction with each of specific coformers, i.e., α-resorcylic acid, gentisic acid, nicotinamide and 3-hydroxybenzoic acid, among many coformers. Further studies have been made, and it is also found that (3S)-3-[2-(6-amino-2-fluoropyridin-3-yl)-4-fluoro-1lH-imidazol-5-yl]-7-[5-chloro-2-(1H-tetrazol-1-yl)phenyl]-2,3-dihydroindolizin-5(1H)-one-3-hydroxybenzoic acid (1/1) (also simply referred to as a "compound A" or "the compound of the invention", hereinafter) is superior in low hygroscopicity and/or oral absorption. These findings lead to the accomplishment of the present invention.

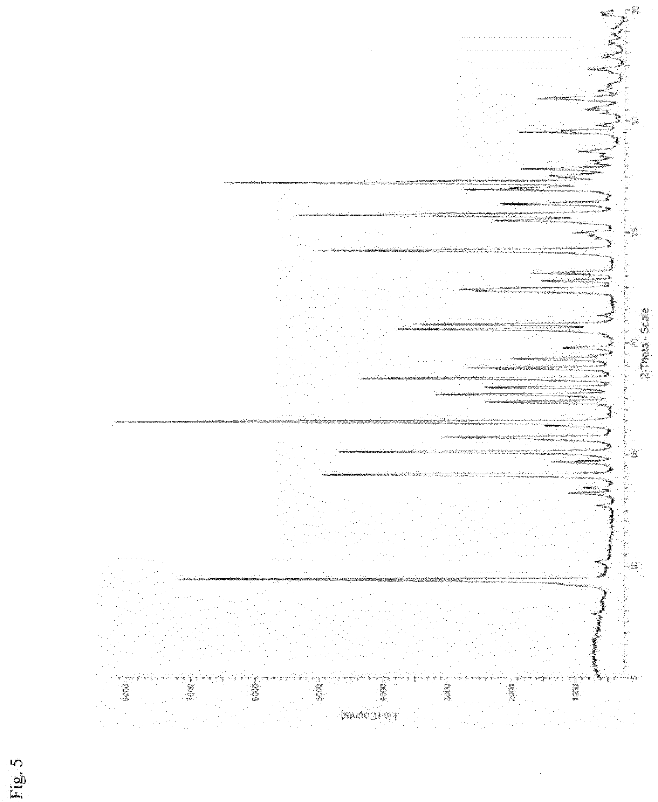

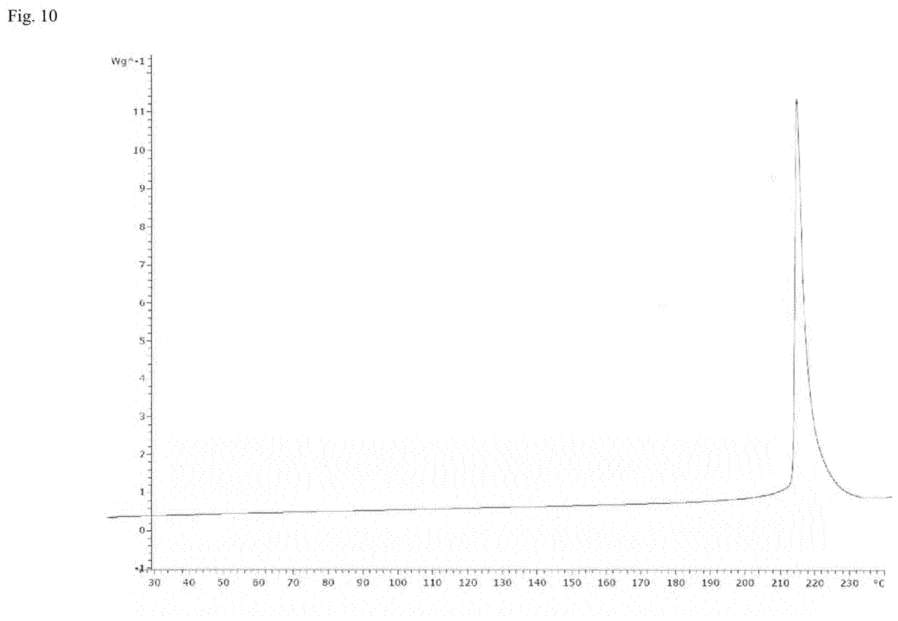

The present invention provides the following embodiments, for example.
[1] (3S)-3-[2-(6-Amino-2-fluoropyridin-3-yl)-4-fluoro-1H-imidazol-5-yl]-7-[5-chloro-2-(1H-tetrazol-1 -yl)phenyl]-2,3-dihydroindolizin-5(1H)-one-3-hydroxybenzoic acid (1/1);
[2] the compound according to the above item [1], wherein the compound is in a crystal form;
[3] the compound according to the above item [2], wherein the crystal form has at least two diffraction peaks at diffraction angles (2θ) of about 9.4, about 14.1, about 15.8, about 16.5, about 17.4, about 18.4, about 20.6 and about 22.4 degrees in a powder X-ray diffraction spectrum;
[3-1] the compound according to the above item [2], wherein the crystal form has at least three diffraction peaks at diffraction angles (2θ) of about 9.4, about 14.1, about 15.8, about 16.5, about 17.4, about 18.4, about 20.6 and about 22.4 degrees in a powder X-ray diffraction spectrum;
[3-2] the compound according to the above item [2], wherein the crystal form has at least four diffraction peaks at diffraction angles (2θ) of about 9.4, about 14.1, about 15.8, about 16.5, about 17.4, about 18.4, about 20.6 and about 22.4 degrees in a powder X-ray diffraction spectrum;
[3-3] the compound according to the above item [2], wherein the crystal form has at least five diffraction peaks at diffraction angles (2θ) of about 9.4, about 14.1, about 15.8, about 16.5, about 17.4, about 18.4, about 20.6 and about 22.4 degrees in a powder X-ray diffraction spectrum;
[3-4] the compound according to the above item [2], wherein the crystal form has diffraction peaks at diffraction angles (2θ) of about 9.4, about 14.1, about 15.8, about 16.5, about 17.4, about 18.4, about 20.6 and about 22.4 degrees in a powder X-ray diffraction spectrum;
[4] the compound according to the above item [2], wherein the crystal form has diffraction peaks at diffraction angles (2θ) of about 9.4, about 13.3, about 14.1, about 14.7, about 15.1, about 15.8, about 16.5, about 17.4, about 17.7, about 18.0, about 18.4, about 18.9, about 19.3, about 19.8, about 20.6, about 20.9, about 22.4, about 22.8, about 23.2, about 24.2, about 25.0, about 25.5, about 25.8, about 26.3, about 27.2, about 27.6, about 27.9, about 28.6, about 29.5 and about 31.0 degrees in a powder X-ray diffraction spectrum;
[5] the compound according to the above item [2], wherein the crystal form is characterized by having a powder X-ray diffraction spectrum chart shown in Fig. 5;
[6] the compound according to any one of the above items [2] to [5] or [3-1] to [3-4], wherein the crystal form has an onset temperature of about 214°C or an exothermic peak temperature of about 215°C in differential scanning calorimetry;
[7] the compound according to the above item [6], wherein the crystal form is characterized by having a differential scanning calorimetry chart shown in Fig. 10;
[8] the compound according to any one of the above items [1] to [7] or [3-1] to [3-4], wherein the compound is a cocrystal;
[9] a pharmaceutical composition containing the compound according to any one of the above items [1] to [8] or [3-1] to [3-4];
[10] the pharmaceutical composition according to the above item [9], wherein the pharmaceutical composition is a blood coagulation factor XIa inhibitor;
[11] the pharmaceutical composition according to the above item [9], wherein the pharmaceutical composition is a prophylactic and/or therapeutic agent for a blood coagulation factor XIa-related disease;
[12] the pharmaceutical composition according to the above item [11], wherein the blood coagulation factor XIa-related disease is a thromboembolic disease;
[13] the pharmaceutical composition according to the above item [12], wherein the thromboembolic disease is arterial cardiovascular thromboembolic disorder, venous cardiovascular thromboembolic disorder, arterial cerebrovascular thromboembolic disorder, venous cerebrovascular thromboembolic disorder or thromboembolic disorder in the heart chamber or in the peripheral circulation;
[14] the pharmaceutical composition according to the above item [12], wherein the thromboembolic disease is cerebral stroke, cerebral thrombosis, cerebral embolism, venous thrombosis, venous thromboembolism, deep venous thrombosis, or a thrombosis and/or a thromboembolism induced by a treatment in which blood is exposed on a surface of an artificial object;
[15] a method for preventing and/or treating a thromboembolic disease, the method comprising administrating an effective amount of the compound according to any one of the above items [1] to [8] or [3-1] to [3-4] to a mammal;
[16] the compound according to any one of the above items [1] to [8] or [3-1] to [3-4], which is used for the prevention and/or treatment of a thromboembolic disease;
[17] a use of the compound according to any one of the above items [1] to [8] or [3-1] to [3-4] for the production of a prophylactic and/or therapeutic agent for a thromboembolic disease;
[18] a method for producing the compound according to any one of the above items [1] to [8] or [3-1] to [3-4] using ethyl acetate in the process of the production;
[19] a compound represented by the structural formula: 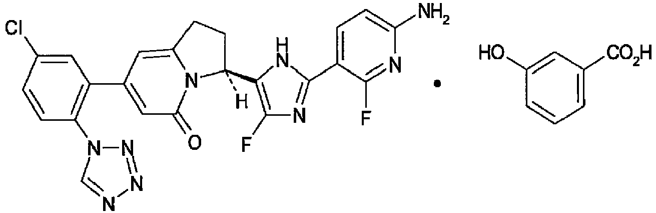
[20] a cocrystal of (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one and 3-hydroxybenzoic acid.
[20-1] (3S)-3-[2-(6-Amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-3-hydroxybenzoic acid (1/1). [20-2] The compound according to the item [20-1], wherein the compound is a crystal.
[20-3] The crystal according to the item [20-2], wherein the crystal has at least two diffraction peaks at diffraction angles (2θ) of about 9.4, about 14.1, about 15.8, about 16.5, about 17.4, about 18.4, about 20.6 and about 22.4 degrees in a powder X-ray diffraction spectrum.
[20-4] The crystal according to the item [20-2], wherein the crystal has diffraction peaks at diffraction angles (2θ) of about 9.4, about 13.3, about 14.1, about 14.7, about 15.1, about 15.8, about 16.5, about 17.4, about 17.7, about 18.0, about 18.4, about 18.9, about 19.3, about 19.8, about 20.6, about 20.9, about 22.4, about 22.8, about 23.2, about 24.2, about 25.0, about 25.5, about 25.8, about 26.3, about 27.2, about 27.6, about 27.9, about 28.6, about 29.5 and about 31.0 degrees in a powder X-ray diffraction spectrum.
[20-5] The crystal according to the item [20-2], wherein the crystal has a powder X-ray diffraction spectrum chart shown in Fig. 5.
[20-6] The crystal according to the item [20-2] or [20-3], wherein the crystal has an onset temperature of about 214°C or an exothermic peak temperature of about 215°C in differential scanning calorimetry.
[20-7] The crystal according to the item [20-6], wherein the crystal has a differential scanning calorimetry chart shown in Fig. 10.
[20-8] The compound according to the item [20-1], wherein the compound is a cocrystal.
[20-9] The crystal according to any one of the items [20-1] to [20-7], wherein the crystal is a cocrystal.
[20-10] A pharmaceutical composition containing the compound according to the item [20-1] or a crystal according to the item [20-2].
[20-11] The pharmaceutical composition according to the item [20-10], wherein the pharmaceutical composition is a blood coagulation factor XIa inhibitor.
[20-12] The pharmaceutical composition according to the item [20-10], wherein the pharmaceutical composition is a prophylactic and/or therapeutic agent for a blood coagulation factor XIa-related disease.
[20-13] The pharmaceutical composition according to the item [20-12], wherein the blood coagulation factor XIa-related disease is a thromboembolic disease.
[20-14] The pharmaceutical composition according to the item [20-13], wherein the thromboembolic disease is arterial cardiovascular thromboembolic disorder, venous cardiovascular thromboembolic disorder, arterial cerebrovascular thromboembolic disorder, venous cerebrovascular thromboembolic disorder or a thromboembolic disorder in the heart chamber or in the peripheral circulation.
[20-15] The pharmaceutical composition according to the item [20-13], wherein the thromboembolic disease is cerebral stroke, cerebral thrombosis, cerebral embolism, venous thrombosis, venous thromboembolism, deep venous thrombosis, or a thrombosis and/or a thromboembolism induced by a treatment in which blood is exposed on a surface of an artificial object.

### ADVANTAGEOUS EFFECTS OF INVENTION

The compound A can be formed into a crystal thereof, and is therefore useful as an active pharmaceutical ingredient having excellent low hygroscopicity and/or oral absorption.

### BRIEF DESCRIPTION OF DRAWINGS

Fig. 1 shows a powder X-ray diffraction spectrum chart of a compound of Example 1(11) (wherein the vertical axis indicates an intensity (counts) and the horizontal axis indicates 2θ (degree)).
Fig. 2 shows a powder X-ray diffraction spectrum chart of a compound of Example 2 (wherein the vertical axis indicates an intensity (counts) and the horizontal axis indicates 2θ (degree)).
Fig. 3 shows a powder X-ray diffraction spectrum chart of a compound of Example 3 (wherein the vertical axis indicates an intensity (counts) and the horizontal axis indicates 2θ (degree)).
Fig. 4 shows a powder X-ray diffraction spectrum chart of a compound of Example 4 (wherein the vertical axis indicates an intensity (counts) and the horizontal axis indicates 2θ (degree)).
Fig. 5 shows a powder X-ray diffraction spectrum chart of a compound of Example 5 (wherein the vertical axis indicates an intensity (counts) and the horizontal axis indicates 2θ (degree)).
Fig. 6 shows a differential scanning calorimetry (DSC) chart of the compound of Example 1(11) (wherein the vertical axis indicates a heat flux (W/g) and the horizontal axis indicates a temperature (°C)).
Fig. 7 shows a differential scanning calorimetry (DSC) chart of the compound of Example 2 (wherein the vertical axis indicates a heat flux (W/g) and the horizontal axis indicates a temperature (°C)).
Fig. 8 shows a differential scanning calorimetry (DSC) chart of the compound of Example 3 (wherein the vertical axis indicates a heat flux (W/g) and the horizontal axis indicates a temperature (°C)).
Fig. 9 shows a differential scanning calorimetry (DSC) chart of the compound of Example 4 (wherein the vertical axis indicates a heat flux (W/g) and the horizontal axis indicates a temperature (°C)).
Fig. 10 shows a differential scanning calorimetry (DSC) chart of the compound of Example 5 (wherein the vertical axis indicates a heat flux (W/g) and the horizontal axis indicates a temperature (°C)).
Fig. 11 shows a thermogravimetry (TG) chart of the compound of Example 1(11) (wherein in the horizontal axis, the vertical axis indicates a rate of change in weight (Weight (%)) and the horizontal axis indicates a temperature (Temperature (°C)).
Fig. 12 shows a thermogravimetry (TG) chart of the compound of Example 2 (wherein in the horizontal axis, the vertical axis indicates a rate of change in weight (Weight (%)) and the horizontal axis indicates a temperature (Temperature (°C)).
Fig. 13 shows a thermogravimetry (TG) chart of the compound of Example 3 (wherein in the horizontal axis, the vertical axis indicates a rate of change in weight (Weight (%)) and the horizontal axis indicates a temperature (Temperature (°C)).
Fig. 14 shows a thermogravimetry (TG) chart of the compound of Example 4 (wherein in the horizontal axis, the vertical axis indicates a rate of change in weight (Weight (%)) and the horizontal axis indicates a temperature (Temperature (°C)).
Fig. 15 shows a thermogravimetry (TG) chart of the compound of Example 5 (wherein in the horizontal axis, the vertical axis indicates a rate of change in weight (Weight (%)) and the horizontal axis indicates a temperature (Temperature (°C)).
Fig. 16 shows isothermal adsorption curves of the compound of Example 1(11) (dihydrate), the compound of Example 2 (RES), the compound of Example 3 (GEN), the compound of Example 4 (NIA), and the compound of Example 5 (3HBA) (wherein the vertical axis indicates a change in dry mass (%) and the horizontal axis indicates a relative humidity (RH) (%)).
Fig. 17 shows the changes in concentrations in blood of cocrystals of the compound of Example 1(11) (dihydrate), the compound of Example 4 (NIA) and the compound of Example 5 (3HBA) when each of the cocrystals is administrated orally to monkeys (1 mg/kg) (wherein the vertical axis indicates a concentration of a compound in plasma (ng/mL) and the horizontal axis indicates a time (h)).

### DESCRIPTION OF EMBODIMENTS

The present invention will be described in detail hereinbelow.

In the present invention, (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one refers to a compound represented by the following structural formula: 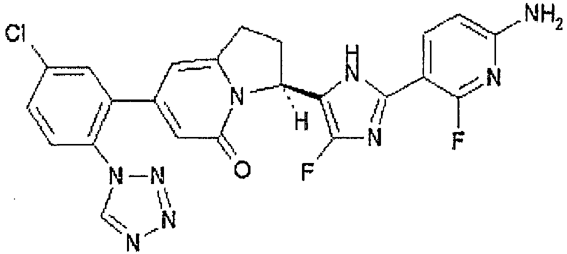 (wherein the symbol: 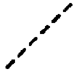 represents an α-configuration, and the symbol: 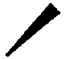 represents a β-configuration).

The compound represented by the above-mentioned structural formula can also be named "(3S)-3-[2-(6-amino-2-fluoro-3-pyridinyl)-4-fluoro-1H-imidazol-5-yl]-7-[5-chloro-2-(1H-tetrazol-1-yl)phenyl]-2,3-dihydro-5(1H)-indolizinone", as described in Example 2(10) in Patent Literature 1.

In the present invention, (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-3-hydroxybenzoic acid (1/1) refers to a compound represented by the following structural formula: 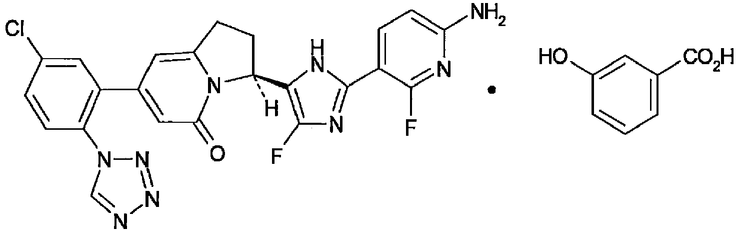 (wherein all symbols are as described above).

The compound represented by the above-mentioned structural formula can also be named:
"(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-3-hydroxybenzoic acid (1/1)",
"(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-3-hydroxybenzoic acid (1 : 1)",
"(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one mono-3-hydroxybenzoate",
"(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one 3-hydroxybenzoic acid (1/1)",
"(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one 3-hydroxybenzoic acid (1 : 1)",
"(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-mono-3-hydroxybenzoate",
"(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one •3-hydroxybenzoic acid (1/1)",
"(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one •3-hydroxybenzoic acid (1 : 1)",
"(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one •mono-3-hydroxybenzoate",
"(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-3-hydroxybenzoic acid",
"(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one 3-hydroxybenzoic acid",
"(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one •3-hydroxybenzoic acid",
"(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one3-hydroxybenzoic acid", or
"(3S)-3-[2-(6-Amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one mono-3-hydroxybenzoate".

In the indication of the compound names, the hyphen (-) located between "(3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one" and "3-hydroxybenzoic acid" may also be expressed by an em dash or a double hyphen (--) instead, as shown in Table 1 below.

**[Table 1]**

| |
|---|
| (3S)-342-(6-Amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-3-hydroxybenzoic acid (1/1) |
| (3S)-3-[2-(6-Amino-2-fluoropyridin-3-yl)4-fluoro-1H-imidazol-5-yl]-7-[5-chloro-2-(1H-tetrazol-1-yl)phenyl]-2,3-dihydroindolizin-5(1H)-one-3-hydroxybenzoic acid (1/1) |
| (3S)-3-[2-(6-Amino-2-fluoropyridin-3-yl)-4-fluoro-1H-imidazol-5-yl]-7-[5-chloro-2-(1H-tetraxol-1-yl)phenyl]-2,3-dihydroindolizin-5(1H)-one-3-hydroxybenzoic acid (1/1) |
| (3S)-3-[2-(6-Amino-2-fluoropyridin-3-yl)4-fluoro-1H-imidazol-5-yl]-7-[5-chloro-2-(1H-tetrazol-1-yl)phenyl]-2,3-dihydroindolizin-5(1H)-one--3-hydroxybenzoic acid (1/1) |

One form of the compound A is, for example, an adduct. The term "adduct" as used herein refers to a novel chemical species (AB) formed by the direct bonding of two different molecules (A) and (B) to each other in such a manner that the number of atoms in either one of the compounds cannot be decreased or increased. Examples of the adduct include a salt and a cocrystal.

In one embodiment, the compound A is an adduct of (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one and 3-hydroxybenzoic acid.

In one embodiment, the compound A is a cocrystal of (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one and 3-hydroxybenzoic acid. The compound A can be characterized by at least one of physical chemical data (a) and (b) shown below. It is preferred that the compound A is characterized by both of physical chemical data (a) and (b). (a) The compound A has (i) a powder X-ray diffraction spectrum shown in Fig. 5, (ii) diffraction angles (2θ) of the powder X-ray diffraction spectrum shown in Fig. 5, (iii) a powder X-ray diffraction spectrum having substantially the same diffraction angles (2θ) (about 9.4, about 13.3, about 14.1, about 14.7, about 15.1, about 15.8, about 16.5, about 17.4, about 17.7, about 18.0, about 18.4, about 18.9, about 19.3, about 19.8, about 20.6, about 20.9, about 22.4, about 22.8, about 23.2, about 24.2, about 25.0, about 25.5, about 25.8, about 26.3, about 27.2, about 27.6, about 27.9, about 28.6, about 29.5 and about 31.0 degrees) as those shown in Table 6, (iv) a powder X-ray diffraction spectrum having at least one, two, three, four, five or more than 5 peaks at a diffraction angle (2θ) or diffraction angles (2θ) selected from substantially the same diffraction angles (2θ) as those shown in Table 6, or (v) a powder X-ray diffraction spectrum having at least one, two, three, four, five or more than five peaks at an a diffraction angle (2θ) or diffraction angles (2θ) selected from about 9.4, about 14.1, about 15.8, about 16.5, about 17.4, about 18.4, about 20.6 and about 22.4 degrees; and (b) the compound A has differential scanning calorimetry (DSC) shown in Fig. 10 or an exothermic peak having an onset temperature of about 214°C or an exothermic peak temperature of about 215°C.

### [Labeling with isotope]

In the compound A, at least one atom may be substituted by an isotope. The compound A labeled with an isotope, e.g., the compound A having a radioisotope incorporated therein, is useful in histological distribution studies on drugs and/or substrates and the like. Examples of the isotope include ²H, ³H, ¹¹C, ¹³C, ¹⁴C, ¹³N, ¹⁵N, ¹⁵O, ¹⁷O, ¹⁸O, ³⁵S, ¹⁸F, ³⁶Cl, ¹²³I and ¹²⁵I.

### [Toxicity]

The toxicity of the compound A is low. Therefore, the compound A can be used safely.

### [Application to pharmaceuticals]

The compound A has a potent FXIa inhibitory activity. Accordingly, the compound of the present invention is useful for preventing and/or treating thromboembolic disease, for example, arterial cardiovascular thromboembolic disorder, venous cardiovascular thromboembolic disorder, arterial cerebrovascular thromboembolic disorder, venous cerebrovascular thromboembolic disorder and thromboembolic disorder in the heart chamber or in the peripheral circulation.

Examples of the arterial cardiovascular thromboembolic disorder include coronary artery disease, ischemic cardiomyopathy, acute coronary syndrome, coronary artery thrombosis, ischemic complications of unstable angina and non-Q wave myocardial infarction, ST-segment-elevation-type and/or non-ST-segment-elevation-type acute myocardial infarction which is medically managed or involves percutaneous coronary intervention, angina pectoris such as stable (exercise-induced) angina pectoris, variant angina pectoris, unstable angina pectoris, myocardial infarction (such as initial myocardial infarction and recurrent myocardial infarction), acute myocardial infarction, reocclusion and stenosis of a blood vessel after coronary artery bypass graft surgery, reocclusion and stenosis after percutaneous transluminal angioplasty, cardiac/transcoronary stent implantation and after thrombolytic therapy for coronary artery, ischemic sudden death and the like.

Examples of the venous cardiovascular thromboembolic disease include deep venous thrombosis (DVT) and/or pulmonary embolism (PE) in major general surgery, abdominal surgery, artificial hip replacement arthroplasty, knee replacement arthroplasty, hip fracture surgery, multiple bone fracture, multiple trauma, traumatic injury, spinal cord injury, burn injury or at the time of entering critical care unit, DVT and/or PE in a patient with acute medical disease with a significantly limited physical activity, DVT and/or PE in a patient receiving cancer chemotherapy, DVT and/or PE in a patient with cerebral stroke, symptomatic or asymptomatic DVT regardless of the presence/absence of PE and the like.

Examples of the arterial cerebrovascular thromboembolic disorder include cerebral stroke, ischemic stroke, the acute phase of cerebral infarction, cerebral stroke in a patient with nonvalvular atrial fibrillation or valvular atrial fibrillation, cerebral arterial thrombosis, cerebral infarction, transient ischemic attack (TIA), lacunar infarct, atherothrombotic cerebral infarction, Branch atheromatous disease (BAD), cerebral arterial embolism, cerebral thrombosis, cerebrovascular disorder, asymptomatic cerebral infarction, and cerebrovascular dementia.

Examples of the venous cerebrovascular thromboembolic disorder include intracranial venous thrombosis, cerebral embolism, cerebral thrombosis, cerebral venous sinus thrombosis, intracranial venous sinus thrombosis, cavernous sinus thrombosis and the like.

Examples of the thromboembolic disease in the heart chamber or in the peripheral circulation include venous thrombosis, systemic venous thromboembolism, recurrent venous thromboembolism, thrombophlebitis, nonvalvular and valvular atrial fibrillation, cardiogenic embolism, disseminated intravascular coagulation (DIC), sepsis, acute respiratory distress syndrome (ARDS), acute lung injury (ALI), chronic obstructive pulmonary disease, antiphospholipid antibody syndrome, liver embolism, hepatic veno-occlusive disease (VOD), kidney embolism, renal vein thrombosis, renal artery occlusion, refractory nephrotic syndrome due to membranous nephropathy or focal sclerosing glomerulonephritis, splenic vein thrombosis, superior mesenteric arterial occlusion, portal vein thrombosis, retinal vein occlusion, atherosclerosis, atherothrombosis, peripheral arterial occlusive disease (PAOD), peripheral arterial disease, arterial embolism, diabetes and metabolic syndrome as well as sequelae thereof, and thrombosis induced by a treatment in which blood is exposed to the surface of an artificial object (such as a medical implant, a medical device, a catheter, a stent, a prosthetic cardiac valve, a heart-lung machine, and a hemodialyzer) which can promote thrombus formation and the like.

Preferable examples of the thromboembolic disease include coronary artery disease, unstable angina, acute coronary syndrome, atrial fibrillation, myocardial infarction(such as initial myocardial infarction and recurrent myocardial infarction), ischemic sudden death, transient ischemic attack, cerebral stroke, peripheral arterial disease, atherosclerosis, peripheral occlusive arterial disease, venous thrombosis, venous thromboembolism, deep venous thrombosis, thrombophlebitis, arterial embolism, coronary artery thrombosis, cerebral arterial thrombosis, cerebral embolism, kidney embolism, portal vein thrombosis, pulmonary embolism, pulmonary infarction, liver embolism, hepatic veno-occlusive disease (VOD)/sinusoidal obstruction syndrome (SOS), thrombotic microangiopathy (TMA), disseminated intravascular coagulation (DIC), sepsis, acute respiratory distress syndrome (ARDS), acute lung injury (ALI), antiphospholipid antibody syndrome, thrombosis due to coronary artery bypass graft surgery, and thrombosis induced by a treatment in which blood is exposed to the surface of an artificial object (such as a medical implant, a medical device, a catheter, a stent, a prosthetic cardiac valve, a heart-lung machine, and a hemodialyzer) which can promote thrombus formation.

In the present specification, atrial fibrillation, atherosclerosis or sepsis includes thromboembolic disease induced by atrial fibrillation, atherosclerosis or sepsis.

More preferable examples of the thromboembolic disease include cerebral stroke, cerebral thrombosis, cerebral embolism, venous thrombosis, venous thromboembolism, deep vein thrombosis, and thrombosis and / or thromboembolism induced by a treatment in which blood is exposed to the surface of an artificial object.

The venous thromboembolism (VTE) includes deep venous thrombosis (DVT), pulmonary embolism (PE) and pulmonary embolism which involves deep venous thrombosis. The prevention and/or treatment of the VTE includes the treatment and/ or the suppression of recurrence of deep vein thrombosis and pulmonary thromboembolism, the onset inhibition of VTE in a patient receiving an orthopedic surgery of lower extremity (such as total knee replacement arthroplasty, total hip replacement and operation of hip fracture), the onset inhibition of DVT and/or PE in a patient with acute medical disease with significantly limited physical activity, the intraoperative and/or postoperative onset inhibition of VTE in a patient receiving abdominal surgery and the onset inhibition of DVT and/or PE in a patient receiving cancer chemotherapy.

The prevention and/or treatment of the ischemic stroke includes the onset inhibition and/or treatment of ischemic stroke and systemic embolism in a patient with nonvalvular atrial fibrillation, the onset inhibition and/or treatment of recurrent cerebral stroke and systemic embolism in a patient with embolic stroke of undetermined source (ESUS), the onset inhibition and/or treatment of ischemic stroke and systemic embolism in a patient with atrial fibrillation associated with acute coronary syndrome (ACS), the onset inhibition and/or treatment of ischemic stroke and systemic embolism in a patient with atrial fibrillation with chronic kidney disease (CKD) or end-stage renal disease, the onset inhibition and/or treatment of Branch atheromatous disease (BAD), and the inhibition of recurrence and/or treatment of ischemic stroke(excepting cardiogenic embolism).

The prevention and/or treatment of the thromboembolic disease induced by the treatment in which blood is exposed to an artificial surface which promotes thrombus formation includes the prevention and/or treatment of thromboembolic disease in a patient receiving prosthetic replacement, the prevention and/or treatment of thromboembolic disease in a patient with installation of a ventricular assist device such as an implantable ventricular assist device, a total replacement type ventricular assist device, a percutaneous ventricular assist device and an extracorporeal ventricular assist device and the prevention and/or treatment of thromboembolic disease in a patient with an indwelled coronary artery stent.

The prevention and/or treatment of the acute coronary syndrome (ACS), coronary artery disease or peripheral arterial disease includes the inhibition of a cardiovascular event in a patient with acute coronary syndrome (ACS), the inhibition of a cardiovascular event in a patient with coronary artery disease or peripheral arterial disease and the inhibition of a cardiovascular event in a patient with diabetes with a high cardiovascular risk (more preferably, in a patient with type 2 diabetes).

In addition, the compound A has a plasma kallikrein inhibitory action, and therefore, is useful for preventing and/or treating disease associated with plasma kallikrein.

Examples of the disease associated with plasma kallikrein include retinopathy, diabetic retinopathy, hypertensive retinopathy, proliferative and nonproliferative retinopathy, age-related macular degeneration (AMD), disorder related to the prevention and/or treatment of hematoma or increased vascular permeability, disease related to edema, hereditary angioedema (HAE), diabetic macular edema (DME), clinically significant macular edema (CSME), cystoid macular edema (CME), retinal edema, edema related to neuroglia, cerebral edema, lymphedema, angioedema, traumatic brain injury, hemorrhagic stroke, intracerebral hemorrhage, cerebral aneurysm, arteriovenous malformation, spinal cord injury, ischemia-reperfusion injury, ischemia, cerebral ischemia, pain, disorder accompanied with elements of inflammation, encephalitis, multiple sclerosis, pruritus, arthritis, inflammatory bowel disease, gout, psoriasis, disease related to activation of stellate cells, Alzheimer's disease, Parkinson's disease, amyotrophic lateral sclerosis, Creutzfeldt-Jakob disease, epilepsy, essential hypertension, hypertension related to diabetes or hyperlipidemia, renal failure, chronic kidney disease, heart failure, proteinuria, blood loss during surgery and the like.

Preferable examples of the disease associated with plasma kallikrein include disease related to edema, hereditary angioedema, macular edema, cerebral edema, retinopathy, formation of edema related to ischemia-reperfusion injury as well as blood loss during surgery such as cardiopulmonary bypass and coronary artery bypass grafting.

When the compound A is applied to a pharmaceutical, the compound of the present invention may be used singly, or may also be used in combination with a drug as mentioned below for the purpose of, for example,:
(1) complementation and/or enhancement of the effects of preventing, treating and/or ameliorating symptoms of a disease,
(2) improvement in the kinetics or absorption, and reduction of the dose of the compound A, and/or
(3) reduction of the side effects of the compound A.

When the compound A is used for preventing and/or treating thromboembolic disease, examples of the drug which can be used in combination with the compound A include an anticoagulant agent, an antiplatelet agent, a thrombolytic agent, a fibrinolytic agent, a serine protease inhibitor, an elastase inhibitor, a steroid, and a combination thereof.

Examples of the anticoagulant agent include a thrombin inhibitor, an antithrombin III activator, a heparin cofactor II activator, other FXIa inhibitors, a plasma and/or tissue kallikrein inhibitor, an inhibitor of plasminogen activator inhibitor (PAI-1), an inhibitor of thrombin-activatable fibrinolysis inhibitor (TAFI), a factor VIIa inhibitor, a factor VIIIa inhibitor, a factor IXa inhibitor, a factor Xa inhibitor, a factor XIIa inhibitor, a combination thereof and the like.

Examples of the antiplatelet agent include a GPII/IIIa blocker, a protease-activated receptor (PAR-1) antagonist, a PAR-4 antagonist, a phosphodiesterase III inhibitor, other phosphodiesterase inhibitors, a P2X1 antagonist, a P2Y1 receptor antagonist, a P2Y12 antagonist, a thromboxane receptor antagonist, a thromboxane A2 synthetase inhibitor, a cyclooxygenase-1 inhibitor, a phospholipase D1 inhibitor, a phospholipase D2 inhibitor, a phospholipase D inhibitor, a glycoprotein VI (GPVI) antagonist, a glycoprotein Ib (GPIB) antagonist, a GAS6 antagonist, aspirin, a combination thereof and the like.

The drug to be used in combination with the compound A is preferably an antiplatelet agent.

Preferable examples of the antiplatelet agent include clopidogrel, prasugrel, ticagrelor, cangrelor, elinogrel, cilostazol, sarpogrelate, iloprost, beraprost, limaprost and/or aspirin, a combination thereof and the like.

Preferably, the drug to be used in combination with the compound A is warfarin, unfractionated heparin, low-molecular-weight heparin, enoxaparin, dalteparin, bemiparin, tinzaparin, semuloparin sodium (AVE-5026), danaparoid, a synthesized pentasaccharide, fondaparinux, hirudin, disulfatohirudin, lepirudin, bivalirudin, desirudin, argatroban, aspirin, ibuprofen, naproxen, sulindac, indomethacin, mefenamate, droxicam, diclofenac, sulfinpyrazone, piroxicam, ticlopidine, clopidogrel, prasugrel, ticagrelor, cangrelor, elinogrel, cilostazol, sarpogrelate, iloprost, beraprost, limaprost, tirofiban, eptifibatide, abciximab, melagatran, ximelagatran, dabigatran, rivaroxaban, apixaban, edoxaban, darexaban, betrixaban, TAK-442, a tissue plasminogen activator, a modified tissue plasminogen activator, anistreplase, urokinase, streptokinase, gabexate, gabexate mesilate, nafamostat, sivelestat, sivelestat sodium hydrate, alvelestat (AZD-9668), ZD-8321/0892, ICI-200880, human elafin (tiprelestat), elafin, α1-antitrypsin (A1AT), cortisone, betamethasone, dexamethasone, hydrocortisone, methylprednisolone, prednisolone, triamcinolone or a combination thereof.

In another embodiment, examples of the drug to be used in combination with the compound A include a potassium channel opener, a potassium channel blocker, a calcium channel blocker, an inhibitor of a sodium-hydrogen exchanger, an antiarrhythmic agent, an antiarteriosclerotic agent, an anticoagulant agent, an antiplatelet agent, an antithrombotic agent, a thrombolytic agent, a fibrinogen antagonist, an antihypertensive diuretic, an ATPase inhibitor, a mineralocorticoid receptor antagonist, a phosphodiesterase inhibitor, an antidiabetic agent, a protease inhibitor, an elastase inhibitor, an anti-inflammatory agent, an antioxidant, an angiogenesis-modulating agent, an agent for treating osteoporosis, hormone replacement therapy, a hormone receptor-modulating agent, an oral contraceptive, an anti-obesity drug, an antidepressant drug, an antianxiety agent, an antipsychotic agent, an antiproliferative agent, an antitumor agent, antiulcer and antigastroesophageal reflux agents, a growth hormone agent and/or a growth hormone secretagogue, a thyroid-mimetic, an anti-infective agent, an antiviral agent, an antimicrobial agent, an antifungal agent, a drug for treating hypercholesterolemia/dyslipidemia and therapy for improving lipid profile, preconditioning of simulated ischemia and/or an agent for stunned myocardium, and a combination thereof.

In another embodiment, examples of the drug to be used in combination with the compound A further include an antiarrhythmic agent, an antihypertensive agent, an anticoagulant agent, an antiplatelet agent, a thrombolytic agent, a fibrinolytic agent, a calcium channel blocker, a potassium channel blocker, a cholesterol/lipid-lowering agent, a serine protease inhibitor, an elastase inhibitor, an anti-inflammatory agent, and a combination thereof.

Examples of the antiarrhythmic agent include an IKur inhibitor, an elastase inhibitor, a serine protease inhibitor, a steroid and the like.

Examples of the antihypertensive agent include an ACE inhibitor, an AT-1 receptor antagonist, a β-adrenergic receptor antagonist, an ETA receptor antagonist, a dual ETA/AT-1 receptor antagonist, a vasopeptidase inhibitor and the like.

In a preferable embodiment, examples of the drug to be used in combination with the compound A include an antiplatelet agent and a combination thereof.

The drug to be used in combination with the compound A may be administered in the form of a compounding preparation in which both of the ingredients are compounded in a preparation or may be administered in the form of separate preparations by the same route of administration or different routes of administration. When the separate preparations are administered, the preparations are not necessarily administered concomitantly, but as needed, each of the preparations may be administered with a time difference. In addition, in the case of the administrations with a time difference, the order of administrations is not particularly limited, but may be appropriately adjusted in order to achieve the desired drug efficacy.

The dose of the above-described other drug(s) which is used in combination with the compound A can be appropriately increased or decreased based on the clinically used dose of the drug(s) or a drug similar thereto. In addition, the compounded ratio of the compound of the present invention and other agent(s) can be appropriately adjusted by considering the age and body weight of the subject of administration, the method for administration, the duration of administration, the target disease, the symptom and the like. Approximately 0.01 to 100 parts by weight of other agent(s) may be combined with 1 part by weight of the compound of the present invention. Two or more kinds of other agent(s) may be used. In addition, examples of the other agent(s) include not only those listed above, but also drug(s) having the same mechanism as those listed above. The drug(s) having the same mechanism as those listed above includes not only those which have been found up to now but also those which will be found in future.

The compound A is normally administered systemically or locally, in the form of an oral preparation or a parenteral preparation. Examples of the oral preparation include an oral liquid preparation (such as an elixir, a syrup, a pharmaceutically acceptable liquid agent, a suspension and an emulsion), an oral solid preparation (such as a tablet (including a sublingual tablet and an orally disintegrating tablet), a pill, a capsule (including a hard capsule, a soft capsule, a gelatin capsule and a microcapsule), a powdered agent, a granule and a lozenge) and the like. Examples of the parenteral preparation include a liquid preparation (such as an injection preparation (such as an intravitreal injection preparation, a subcutaneous injection preparation, an intravenous injection preparation, an intramuscular injection preparation, an intraperitoneal injection preparation and a preparation for drip infusion), an eye drop (such as an aqueous eye drop (such as an aqueous ophthalmic solution, an aqueous ophthalmic suspension, a viscous eye drop and a solubilized eye drop) and a non-aqueous eye drop (such as a non-aqueous ophthalmic solution and a non-aqueous ophthalmic suspension))), an external preparation (such as an ointment (such as an ophthalmic ointment)), an ear drop and the like. The above-described preparation may be a controlled-release preparation such as an immediate-release preparation and a sustained release preparation. The above-described preparation can be prepared by a known method, for example, by a method described in Pharmacopeia of Japan or the like.

The oral liquid preparation as an oral preparation is prepared, for example, by dissolving, suspending or emulsifying an active ingredient in a generally used diluent (such as purified water, ethanol and a mixed liquid thereof). In addition, the liquid preparation may further contain a wetting agent, a suspending agent, an emulsifying agent, a sweetening agent, a flavoring agent, a perfume, a preservative, a buffer agent and the like.

The oral solid preparation as an oral preparation is prepared, for example, by mixing an active ingredient with an excipient (such as lactose, mannitol, glucose, microcrystalline cellulose and starch), a bonding agent (such as hydroxypropyl cellulose, polyvinylpyrrolidone and magnesium aluminometasilicate), a disintegrating agent (such as calcium cellulose glycolate), a lubricant (such as magnesium stearate), a stabilizer, a solubilizing agent (such as glutamic acid and aspartic acid) and the like by a routine procedure. In addition, if necessary, the active ingredient may be coated with a coating agent (such as white soft sugar, gelatin, hydroxypropyl cellulose and hydroxypropyl methylcellulose phthalate) or may be coated with two or more layers.

The external preparation as a parenteral preparation is prepared by a known method or according to a normally used formulation. For example, an ointment is prepared by triturating or melting an active ingredient in a base. An ointment base is selected from those which are known and those which are normally used. For example, one selected from the followings is used or two or more kinds selected from the followings are used by being mixed together: a higher fatty acid or a higher fatty acid ester(such as adipic acid, myristic acid, palmitic acid, stearic acid, oleic acid, an adipate, a myristate, a palmitate, a stearate and an oleate), waxes (such as beeswax, whale wax and ceresin), a surface-active agent (such as a polyoxyethylene alkyl ether phosphoric ester), a higher alcohol (such as cetanol, stearyl alcohol and cetostearyl alcohol), a silicone oil (such as dimethyl polysiloxane), hydrocarbons (such as hydrophilic petrolatum, white petrolatum, purified lanolin and liquid paraffin), glycols (such as ethylene glycol, diethylene glycol, propylene glycol, polyethylene glycol and macrogol), a vegetable oil (such as castor oil, olive oil, sesame oil and turpentine oil), an animal oil (such as mink oil, egg-yolk oil, squalane and squalene), water, an absorption promoter and an agent for preventing skin rash. In addition, a moisturizing agent, a preservative, a stabilizing agent, an antioxidant, a flavoring agent and the like may be contained.

The injection preparation as a parenteral preparation includes a solution, a suspension, an emulsion and a solid injection preparation which is used at the time of use by being dissolved or suspended in a solvent. The injection preparation is used, for example, by dissolving, suspending or emulsifying an active ingredient in a solvent. Examples of the solvent used include distilled water for injection, saline, a vegetable oil, alcohols such as propylene glycol, polyethylene glycol and ethanol and the like as well as a mixture thereof. In addition, the injection preparation may contain a stabilizer, a solubilizing agent (such as glutamic acid, aspartic acid and polysorbate 80 (registered trademark)), a suspending agent, an emulsifying agent, an analgesic, a buffer agent, a preservative and the like. The above-described injection preparation is prepared by being sterilized at the final process or by an aseptic manipulation method. In addition, the above-described injection preparation can be also used by preparing a sterile solid preparation, for example, a lyophilized preparation, and dissolving the sterile solid preparation in sterilized or sterile distilled water for injection or other solvent before use of the preparation.

In order to use the compound A or a combination of the compound A and other drug for the above-described purpose, the compound A or the combination is normally administered systemically or locally, in the form of an oral preparation or a parenteral preparation. The dose varies depending on the age, the body weight, the symptom, the therapeutic effect, the method for administration, the duration of the treatment and the like. However, normally, the dose per adult is in the range of from 1 ng to 1,000 mg per administration, from one to several oral administrations per day or the dose per adult is in the rage of from 0.1 ng to 10 mg per administration, from one to several parenteral administrations per day. Alternatively, the dose is continuously administrated intravenously for a period of time in the range of 1 to 24 hours per day. Of course, the dose varies depending on various factors as described above, and therefore, there are some cases in which a dose below the above-described dose is sufficient and there are other cases in which administration of a dose which exceeds the above-described range is required.

In the present invention, (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one, or a salt, solvate, cocrystal or the like thereof can be produced by the method mentioned in the section "EXAMPLES" below or a method similar to the method. When recrystallization is carried out, a seed crystal may be used or may not be used.

### EXAMPLES

The present invention will be described in detail by referring to Examples hereinbelow, but the present invention is not limited to Examples.

Concerning chromatographic separation or TLC, a solvent in parentheses corresponds to an eluting solvent or a developing solvent employed and a ratio is expressed by volume ratio.

Concerning NMR, a solvent in parentheses corresponds to a solvent used for the measurement.

A compound name used in the present specification is given by using a computer program ACD/Name (registered trademark) of Advanced Chemistry Development which generally denominates a compound according to the IUPAC nomenclature or by denomination according to the IUPAC nomenclature.

The measuring time, solvents and column conditions used for LC/MS analyses in the following Examples are shown hereinbelow. Meanwhile, t_{R} means Retention time. Condition a. column YMC-Triart C18, 2.0 mm x 30 mm, 1.9 µm; column temperature 30°C; mobile phase (Liquid A) 0.1% trifluoroacetic acid aqueous solution and (Liquid B) 0.1% trifluoroacetic acid-acetonitrile solution; flow rate 1.0 mL/min; analysis time 1.5 minutes; gradient: 0 minute (Liquid A/Liquid B = 95/5), 0.1 minutes (Liquid A/Liquid B = 95/5), 1.2 minutes (Liquid A/Liquid B = 5/95), 1.4 minutes (Liquid A/Liquid B = 5/95), 1.41 minutes (Liquid A/Liquid B = 95/5), 1.5 minutes (Liquid A/Liquid B = 95/5)
Condition b. column Waters ACQUITY UPLC (registered trademark) BEH C18, 2.1 mm x 30 mm, 1.7 µm; column temperature 40°C; mobile phase (Liquid A) 0.1% formic acid aqueous solution and (Liquid B) 0.1% formic acid-acetonitrile solution; flow rate 1.0 mL/min; analysis time 1.5 minutes; gradient: 0 minute (Liquid A/Liquid B = 95/5), 0.1 minutes (Liquid A/Liquid B = 95/5), 1.2 minutes (Liquid A/Liquid B = 5/95), 1.4 minutes (Liquid A/Liquid B = 5/95), 1.41 minutes (Liquid A/Liquid B = 95/5), 1.5 minutes (Liquid A/Liquid B = 95/5).

### [Experimental Examples]

### Example 1 (1): 6-Fluoro-5-iodo-2-pyridinamine

N-iodosuccinimide (56.5 g) was added in multiple portions (3 portions) to a solution of 6-fluoro-2-pyridinamine (25.6 g) in N,N-dimethylformamide (200 mL) under ice cooling. The mixture was stirred at room temperature for 3 hours, and thereafter, to the reaction liquid, city water (0.5 L) was added. The mixture was extracted three times with ethyl acetate/hexane (1/1, 300 mL), and the organic layer was washed with saturated sulfurous acid aqueous solution (0.5 L), saturated sodium carbonate aqueous solution (0.5 L, twice), city water (0.5 L) and saturated saline (0.5 L), was dried, and thereafter, was concentrated. To the obtained residue, hexane/ethyl acetate (3/1, 150 mL) was added, and the slurry was washed at room temperature, and was filtrated. The obtained solid was dried to give the title compound (36.7 g) having the following physical property.
TLC: Rf 0.56 (ethyl acetate : hexane = 1 : 2).

### Example 1 (2): Bis(2-methyl-2-propanyl) (6-fluoro-5-iodo-2-pyridinyl)imidodicarbonate

To a solution of the compound (36.7 g) produced in Example 1(1) and 4-dimethylaminopyridine (0.9 g) in acetonitrile (300 mL), a solution of di-tert-butyl decarbonate (74.0 g) in acetonitrile (100 mL) was added. The resultant mixture was stirred at room temperature for 3 hours. The reaction solution was concentrated, and the obtained residue was dissolved in ethyl acetate (500 mL), and the mixture was washed with saturated ammonium chloride aqueous solution (400 mL), and the aqueous layer was extracted with ethyl acetate (200 mL). The combined organic layers were dried, and thereafter, were concentrated. The obtained residue was purified by silica gel column chromatography (ethyl acetate : hexane = 5 : 95 to 10 : 90) to give the title compound (45.06 g) having the following physical properties.
¹H-NMR (CDCl₃): δ 8.14 (t, 1H), 7.03 (dd, 1H), 1.47 (s, 18H).

### Example 1 (3): 2-methyl-2-propanyl (5-cyano-6-fluoro-2-pyridinyl)carbamate

A solution of the compound (9.1 g) produced in Example 1(2), zinc (II) cyanide (7.32 g) and tetrakis(triphenylphosphine)palladium (0) (1.2 g) in 1-methyl-2-pyrrolidinone (60 mL) was deaerated under reduced pressure. Under microwave irradiation, the mixture was stirred at 130°C for 1 hour, and thereafter, was left to cool. The reaction solution was diluted with ethyl acetate (100 mL), and thereafter, was filtrated through Celite to remove insoluble matters, and the insoluble matters were washed with ethyl acetate (50 mL). The filtrate was subjected to liquid separation, and the aqueous layer was extracted again with ethyl acetate (100 mL). The organic layers were combined, were dried, and thereafter, were concentrated. The obtained residue was purified by silica gel column chromatography (ethyl acetate : hexane = 5 : 95 to 80 : 20) to give the title compound (2.1 g) having the following physical property.
TLC: Rf 0.25 (ethyl acetate : hexane = 10 : 90).

### Example 1 (4): 2-methyl-2-propanyl [6-fluoro-5-(N-hydroxycarbamimidoyl)-2-pyridinyl]carbamate

To a solution of the compound (1.56 g) produced in Example 1(3) and hydroxylamine hydrochloride (0.91 g) in ethanol (40 mL), N,N-diisopropylethylamine (2.84 mL) was added. The resultant mixture was stirred at 40°C overnight. The reaction solution was concentrated, and the obtained residue was dissolved in ethyl acetate (50 mL). To the mixture, city water (50 mL) was added to wash, and thereafter, the organic layer was dried, and thereafter, was concentrated to give the crude title compound (1.93 g) having the following physical properties.
LC/MS t_{R} 0.60 minutes; MS (ES+) m/z 271 (M+H) (Condition a).

### Example 1 (5): 2-methyl-2-propanyl (5-carbamimidoyl-6-fluoro-2-pyridinyl)carbamate acetate

To a solution of the compound (1.93 g) produced in Example 1(4) in acetic acid (10 mL), acetic acid anhydride (0.75 mL) was added. The resultant mixture was stirred at room temperature for 1 hour. To the reaction liquid, palladium (II) hydroxide (20%, 250 mg) was added, and the mixture was stirred under hydrogen atmosphere at room temperature for 3 hours. The reaction liquid was filtrated through Celite, and the filtrate was concentrated under reduced pressure to give the crude title compound (2.99 g) having the following physical properties.
LC/MS t_{R} 0.59 minutes; MS (ES+) m/z 255 (M+H) (Condition a).

### Example 1 (6): 2-methyl-2-propanyl (5-carbamimidoyl-6-fluoro-2-pyridinyl)carbamate hydrochloride

To a solution of the compound (2.6 g) produced in Example 1(5) in methanol (10 mL), a 10% hydrogen chloride/methanol (6.5 mL) solution was added. The resultant mixture was stirred at room temperature for 10 minutes. To the reaction liquid, toluene was added, and the mixture was concentrated to give the crude title compound (2.63 g) having the following physical properties.
LC/MS t_{R} 0.58 minutes; MS (ES+) m/z 255 (M+H) (Condition a).

### Example 1(7): (3S)-3-(chloroacetyl)-7-[5-chloro-2-(1H-tetrazol-1-yl)phenyl]-2,3-dihydro-5(1H)-indolizinone

To a solution of (3S)-7-[5-chloro-2-(1H-1,2,3,4-tetrazol-1-yl)phenyl]-5-oxo-1,2,3,5-tetrahydroindolizine-3-carboxylic acid (described in Example 9 of International Publication No. 2013/093484 pamphlet) (3.0 g) in dichloromethane (15 mL), 1-chloro-N,N,2-trimethyl-1-propen-1-amine (1.33 mL) was added under ice cooling. The resultant mixture was stirred at 0°C for 40 minutes. To the mixture, trimethylsilyldiazomethane (2 M hexane solution, 8.4 mL) was added, and thereafter, the mixture was stirred at 0°C for additional 1 hour. To the mixture, concentrated hydrochloric acid (0.87 mL) was added under ice cooling, and the mixture was stirred at room temperature for 20 minutes. To the reaction liquid, city water (50 mL) was added, and the mixture was extracted twice with dichloromethane (50 mL). The organic layer was dried, and thereafter, was concentrated, and the obtained residue was purified by silica gel column chromatography (ethyl acetate : hexane = 40 : 60 to 100 : 0) to give the title compound (2.32 g) having the following physical properties.
LC/MS t_{R} 0.80 minutes; MS (ES+) m/z 390 (M+H) (Condition a).

### Example 1(8): 2-methyl-2-propanyl[5-(5-{7-[5-chloro-2-(1H-tetrazol-1-yl)phenyl]-5-oxo-1,2,3,5-tetrahydro-3-indolizinyl}-1H-imidazol-2-yl)-6-fluoro-2-pyridinyl]carbamate

To a solution of the compound (1.5 g) produced in Example 1(6) and the compound (1.0 g) produced in Example 1(7) in acetonitrile (50 mL), potassium carbonate (0.70 g) was added. The resultant mixture was stirred at 80°C for 17 hours. The reaction solution was diluted with ethyl acetate (100 mL), and thereafter, the solution was washed with city water (100 mL) and saturated saline (200 mL), was dried, and thereafter, was concentrated. The obtained residue was purified by silica gel column chromatography (ethyl acetate : hexane = 50 : 50 to 100 : 0, followed by methanol: ethyl acetate = 5 : 95) to give the title compound (1.11 g) having the following physical properties.
LC/MS t_{R} 0.81 minutes; MS (ES+) m/z 590 (M+H) (Condition a).

### Example 1(9): 2-methyl-2-propanyl [5-(5-{(3S)-7-[5-chloro-2-(1H-tetrazol-1-yl)phenyl]-5-oxo-1,2,3,5-tetrahydro-3-indolizinyl}-4-fluoro-1H-imidazol-2-yl)-6-fluoro-2-pyridinyl]carbamate and 2-methyl-2-propanyl[5-(5-{(3R)-7-[5-chloro-2-(1H-tetrazol-1-yl)phenyl]-5-oxo-1,2,3,5-tetrahydro-3-indolizinyl}-4-fluoro-1H-imidazol-2-yl)-6-fluoro-2-pyridinyl]carbamate

To a suspension of the compound (264 mg) produced in Example 1(8) and sodium carbonate (118 mg) in acetonitrile (10 mL)/tetrahydrofuran (5 mL), 1-chloromethyl-4-fluoro-1,4-diazoniabicyclo[2.2.2]octane bis(tetrafluoroborate) (selectfluor (registered trademark)) (95 mg) was added. The resultant mixture was stirred under cooling in an ice/brine bath for 3 hours. The reaction solution was diluted with ethyl acetate (20 mL), and to the solution, sodium sulfite aqueous solution (40 mL) was added. The aqueous layer was extracted twice with ethyl acetate (50 mL), and the combined organic layers were dried, and thereafter, were concentrated. The obtained residue was purified by silica gel column chromatography (aminosilica, ethyl acetate : hexane = 50 : 50 to 100 : 0, followed by methanol: ethyl acetate = 5 : 95) to give the mixture (71.2 mg) of the S-configuration compound and the R-configuration compound of Example 2 (9). The mixture (20 mg) was purified by optical resolution (DAICEL, CHIRALFLASH (registered trademark) IC column, (particle size: 20 µm; column length: 100×30 mm I.D.), flow rate: 24 mL/min; column temperature: room temperature; mobile phase (A): acetonitrile; mobile phase (B): methanol; isocratic (mobile phase (A) : mobile phase (B) = 90 :10), 20 minutes; detector: UV Yamazen UV-254W UV-Detector) to give the title compounds (the S-configuration compound of Example 1(9): 7.9 mg, and the R-configuration compound of Example 1(9): 7.7 mg). In the optical resolution under the above-mentioned conditions, the retention times of the title compounds were 13 minutes (the S-configuration compound of Example 1(9)) and 9.5 minutes (the R-configuration compound of Example 1(9)), respectively.

The physical properties of each of the title compounds when being analyzed under liquid chromatographic conditions in parentheses below are shown hereinbelow. The S-configuration compound of Example 1(9):
LC t_{R} 10.4 minutes (column: DAICEL CHIRALPAK (registered trademark) IC 5 µm 4.6 mm × 250 mm, mobile phase: acetonitrile/methanol = 90/10, flow rate: 1.0 mL/min).
The R-configuration compound of Example 1(9):
LC t_{R} 7.95 minutes (column: DAICEL CHIRALPAK (registered trademark) IC 5 µm 4.6 mm × 250 mm, mobile phase: acetonitrile/methanol = 90/10, flow rate: 1.0 mL/min).

### Example 1(10): (3S)-3-[2-(6-amino-2-fluoro-3-pyridinyl)-4-fluoro-1H-imidazol-5-yl]-7-[5-chloro-2-(1H-tetrazol-1-yl)phenyl]-2,3-dihydro-5(1H)-indolizinone

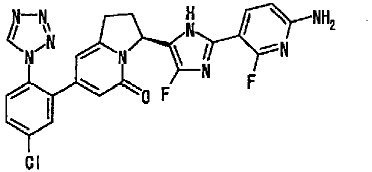

To a suspension of the S-configuration compound (436 mg) of Example 1(9) in ethyl acetate (6 mL), concentrated hydrochloric acid (2 mL) was added. The resultant mixture was stirred at room temperature for 20 minutes. The reaction solution was concentrated under reduced pressure, and the obtained residue was redissolved in tetrahydrofuran (10 mL). A saturated aqueous sodium bicarbonate solution (20 mL) was added to the solution, and the resultant mixture was extracted with ethyl acetate (20 mL, twice). The organic layers were combined, were dried, and thereafter, were concentrated. The obtained residue was purified by silica gel column chromatography (aminosilica, methanol: ethyl acetate = 0 : 100 to 5 : 95) to give the title compound (321 mg) having the following physical properties. In addition, the absolute configuration of this compound was determined by X-ray crystallography using a single crystal of a complex of the compound of this compound and FXIa.
TLC: Rf 0.60 (methanol: ethyl acetate = 5 : 95);
¹H-NMR (CD₃OD): δ 9.31 (s, 1H), 7.91 (dd, 1H), 7.74 - 7.65 (m, 3H), 6.44 (dd, 1H), 6.21 (s, 1H), 6.03 (s, 1H), 5.83 (dd, 1H), 3.39-3.06 (m, 2H), 2.62 - 2.48 (m, 2H);
LC t_{R} 22.5 minutes (column DAICEL CHIRALPAK (registered trademark) IC 5 µm 4.6 mm × 250 mm, mobile phase: hexane/ethyl acetate = 30/70, flow rate: 1.0 mL/min);
[α]²⁵_{D} = +44.1° (CH₃OH, c = 1.00).

### Example 1(11): (3S)-3-[2-(6-amino-2-fluoro-3-pyridinyl)-4-fluoro-1H-imidazol-5-yl]-7-[5-chloro-2-(1H-tetrazol-1-yl)phenyl]-2,3-dihydro-5(1H)-indolizinone dihydrate

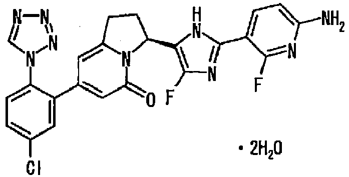

The compound (100 mg) of Example 1(10) was dissolved in acetonitrile (1.0 mL) and water (0.018 mL) while heating at 75°C, the resultant solution was then stirred at 40°C for 2 hours, and was then stirred at room temperature for 30 minutes to generate precipitates, and the precipitates were filtrated out and were then dried under reduced pressure to give the title compound (76 mg) having the following physical property values.
¹H-NMR (CD₃OD): δ 9.31 (s, 1H), 7.91 (dd, 1H), 7.74 - 7.65 (m, 3H), 6.44 (dd, 1H), 6.21 (s, 1H), 6.03 (s, 1H), 5.83 (dd, 1H), 3.39 - 3.06 (m, 2H), 2.62 - 2.48 (m, 2H);
LC/MS t_{R} 0.82 minutes; MS (ES+) m/z 508 (M+H) (Condition a).

### Example 1(12): (3R)-3-[2-(6-amino-2-fluoro-3-pyridinyl)-4-fluoro-1H-imidazol-5-yl]-7-[5-chloro-2-(1H-tetrazol-1-yl)-phenyl]-2,3-dihydro-5(1H)-indolizinone

The same procedure as in Example 1(10) was carried out, except that the R-configuration compound of Example 1(9) was used in place of the S-configuration compound of Example 1(9), thereby giving the title compound having the following physical property values.
¹H-NMR (CD₃OD): δ9.31 (s, 1H), 7.91 (dd, 1H), 7.71 - 7.68 (m, 3H), 6.44 (dd, 1H), 6.20 (s, 1H), 6.03 (s, 1H), 5.84 (dd, 1H), 3.37-3.06 (m, 2H), 2.61 - 2.53 (m, 2H).

### Example 1(13): (3R)-3-[2-(6-Amino-2-fluoro-3-pyridinyl)-4-fluoro-1H-imidazol-5-yl]-7-[5-chloro-2-(1H-tetrazol-1-yl)phenyl]-2,3-dihydro-5(1H)-indolizinone dihydrate

The same procedure as in Example 1(11) was carried out, except that the compound of Example 1(12) was used in place of the compound of Example 1(10), thereby giving the title compound having the following physical property values.
¹H- NMR (DMSO-d6): δ 11.70 (s, 1H), 9.64 (s, 1H), 7.89 - 7.75 (m, 4H), 6.75 (brs, 2H), 6.38 (dd, 1H), 6.01 (d, 1H), 5.92 (s, 1H), 5.69 (dd, 1H), 3.18 - 3.00 (m, 2H), 2.50 - 2.24 (m, 2H).

### [Examination about crystallization]

Examination about the crystallization of the compound of Example 1(10) was carried out. In the following examination, with respect to the experiment in which the optical isomerism did not affect results, the experiment was also carried out using the compound of Example 1(12) or Example 1(13).

Firstly, the possibility of crystallization of a salt of the compound of Example 1(10) was examined using the compound of Example 1(10).

The crystallization of salts of the compound of Example 1(10) was examined by a evaporative concentration method (heating: 40°C, evaporation: room temperature) with an automatic crystallization system ("Core Module (X)", manufactured by Freeslate) using 11 types of acids (hydrochloric acid (1 equivalent), sulfuric acid (1 equivalent), methanesulfonic acid (1 equivalent), 4-toluenesulfonic acid, ethanesulfonic acid (1 equivalent), isethionic acid (1 equivalent), ethanedisulfonic acid (1/2 equivalent), 1,5-naphthalenedisulfonic acid (1/2 equivalent), cyclamic acid (1 equivalent), nitric acid (1 equivalent)) and using 12 types of solvents (methanol, ethanol, 2-propanol, acetone, acetonitrile, ethyl acetate, methyl tert-butyl ether (MTBE), tetrahydrofuran, heptane, toluene, dichloromethane, water).

As the result of this examination, any crystal of the compound of Example 1(10) was not produced under the above-mentioned various conditions.

Next, the possibility of the crystallization of a free form (the compound of Example 1(10)) was examined.

The examination was carried out about the crystallization of a free form using the compound of Example 1(13), also using 51 types of solvents (methanol, ethanol, 2-propanol, acetone, acetonitrile, ethyl acetate, methyl tert-butyl ether (MTBE), tetrahydrofuran, heptane, toluene, dichloromethane, water, 1, 4-dioxane, 1-propanol, 1-butanol, 2-butanol, 2-methyl-1-propanol, ethyl formate, methyl acetate, propyl acetate, isopropyl acetate, fluorobenzene, butyronitrile, methyl ethyl ketone, methyl isobutyl ketone, diisopropyl ether, 1,2-dichloroethane, dimethyl carbonate, pyridine, 1,2-dimethoxyethane, tert-amyl alcohol, cyclohexane, triethylamine, 2,2,2-trifluoroethanol, octane, 2-hexanone, butyl acetate, cyclopentanone, 3-methyl-1-butanol, chlorobenzene, 2-ethoxyethanol, cumene, anisole, N,N-dimethylformamide, mesitylene, dimethylacetamide, dimethyl sulfoxide, ethylene glycol, formamide) and using an automatic crystallization system ("Core Module (X)", manufactured by Freeslate). As the crystallization method, three types of conditions, i.e., a slurry method (25°C and 40°C), a precipitation method (precipitation at room temperature) and an evaporative concentration method (heating at 40°C or 80°C, evaporation at room temperature), were preset, and the solvents and the crystallization methods were combined to set 576 kinds of crystallization conditions for each of the salts.

As the result of this examination, only a dihydrate (Example 1(13)) was obtained as a crystal of Example 1(12) which had satisfactory crystallinity. From this data, it was found that, with respect to the compound of Example 1(10) that was an enantiomer of the compound, only a dihydrate (Example 1(11)) was obtained as a crystal having satisfactory crystallinity, but the compound of Example 1(11) had high hygroscopicity and poor oral absorption as mentioned below and therefore could not be selected as an active ingredient for a pharmaceutical when being in an unmodified form.

Since no crystal which meets the problems could be obtained in the above-mentioned examination about salts and free forms, the examination about the crystallization of a cocrystal of the compound of Example 1(10) was further carried out as follows.

The possibility of crystallization of a cocrystal was examined using the compound of Example 1(13).

The crystallization of a cocrystal was examined using 60 types of reagents (oxalic acid, fumaric acid, adipic acid, L-tartaric acid, D-tartaric acid, benzoic acid, 2-hydroxybenzoic acid, 3-hydroxybenzoic acid, 4-hydroxybenzoic acid, 2,3-dihydroxybenzoic acid, 2,4-dihydroxybenzoic acid, 2,5-dihydroxybenzoic acid, 3,4-dihydroxybenzoic acid, 3,5-dihydroxybenzoic acid, saccharin, orotic acid, 1-hydroxy-2-naphthoic acid, L-pyroglutamic acid, D-pyroglutamic acid, benzamide, ethyl maltol, nicotinamide, glycolic acid, sorbic acid, L-camphoric acid, D-camphoric acid, urea, taurine, malonic acid, L-mandelic acid, D-mandelic acid, maleic acid, anthranilic acid, L-alanine, D-alanine, L-lactamide, D-lactamide, glycine, L-tryptophan, D-tryptophan, N-acetylglycine, L-valine, D-valine, ascorbic acid, citric acid, glutaric acid, erythorbic acid, galactaric acid, L-malic acid, D-malic acid, sorbitol, myo-inositol, glucuronic acid, sebacic acid, succinic acid, D-histidine), also using 6 types of solvents (methanol, acetone, acetonitrile, ethyl acetate, toluene, dichloromethane) by an evaporative concentration method (heating at 40°C, evaporation at room temperature) with an automatic crystallization system ("Core Module (X)", manufactured by Freeslate).

As the result of this examination, crystals of only 4 types of counters, i.e., 3,5-dihydroxybenzoic acid, 2,5-dihydroxybenzoic acid, nicotinamide and 3-hydroxybenzoic acid (also abbreviated as "RES", "GEN", "NIA" and "3HBA", hereinafter), were formed under the above-mentioned various conditions. With respect to 3HBA, 2 types of crystals were formed. As shown below, with respect to RES and GEN among the 4 types of counters, the problem of hygroscopicity was concerned from the physical property data of differential scanning calorimetry and thermogravimetry. In contrast, with respect to each of NIA and 3HBA, there was no concern about this problem and therefore an S-configuration thereof was produced and was subjected to the following experiments.

### Example 2: (3R)-3-[2-(6-Amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-α-resorcylic acid (2/1)

To the compound of Example 1(13) (20 mg), α-resorcylic acid (2.83 mg) and acetonitrile (100 µL) were added. The resultant mixture was stirred at 25°C for 1 day to give precipitates. The precipitates were filtrated out, and were then dried in a draft to give a crystal of the title compound which had the following physical property values.
¹H-NMR (DMSO-d6): 11.70, 9.65, 9.13, 7.86 - 7.75, 7.14 - 7.13, 6.96 - 6.92, 6.78 - 6.75, 6.40 - 6.36, 6.00, 5.92, 5.71, 5.68, 3.32, 3.20 - 2.93, 2.27 - 2.20.

### Example 3: (3R)-3-[2-(6-Amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-gentisic acid (2/1)

To the compound of Example 1(13) (20 mg), gentisic acid (2.83 mg) and acetonitrile (100 µL) were added. The resultant mixture was stirred at 25°C for 1 day to give precipitates. The precipitates were filtrated out and were then dried under reduced pressure to give crystals of the title compound which had the following physical property values. ¹H-NMR (DMSO-d6): 12.67, 11.70, 9.65, 9.53, 7.89 - 7.75, 6.78 - 6.75, 6.40 - 6.37, 6.00, 5.92, 5.71 - 5.68, 3.32, 3.17 - 2.93, 2.26 - 2.20.

### Example 4: (3S)-3-[2-(6-Amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-nicotinamide (2/1)

The compound of Example 1(11) (500 mg) was mixed with nicotinamide (56.1 mg) and acetonitrile (5 mL), and the resultant mixture was stirred at 25°C for 1 day to give precipitates. The precipitates were filtered out and were then dried under reduced pressure to give the title compound title compound (465 mg) having the following physical property values.
¹H-NMR (DMSO-d6): δ11.71, 9.65, 9.11- 8.89, 8.79 - 8.60, 8.24 - 8.09, 7.90 - 7.72, 7.61, 7.53 - 7.44, 6.75, 6.38, 6.00, 5.93, 5.74 - 5.65, 3.20 - 2.93, 2.29 - 2.19.

### Example 5: (3S)-3-[2-(6-Amino-2-fluoropyridin-3-yl)-4-fluoro-1H-imidazol-5-yl]-7-[5-chloro-2-(1H-tetrazol-1-yl)-phenyl]-2,3-dihydroindolizin-5(1H)-one-3-hydroxybenzoic acid (1/1)

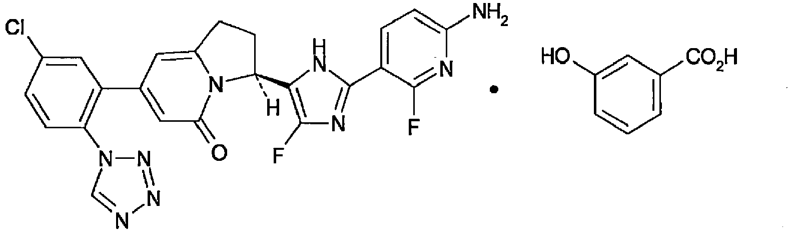

The compound of Example 1(11) (500 mg) was mixed with 3-hydroxybenzoic acid (127.0 mg) and ethyl acetate (5 mL), and the resultant mixture was stirred at 25°C for 1 day to give precipitates. The precipitates were filtered out and were then dried under reduced pressure to give the title compound (529 mg) having the following physical property values. ¹H-NMR (DMSO-d6): 12.82, 11.71, 9.73, 9.65, 7.90 - 7.72, 7.40 - 7.22, 7.02 - 6.93, 6.75, 6.38, 6.00, 5.92, 5.74 - 5.65, 3.20 - 2.93, 2.29 - 2.19.

### Example 6: (3R)-3-[2-(6-Amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-3-hydroxybenzoic acid

(2/1) The compound of Example 1(12) (500 mg) was mixed with 3-hydroxybenzoic acid (63.5 mg) and acetonitrile (5 mL), and the resultant mixture was stirred at 25°C for 1 day to give precipitates. The precipitates were filtered out and were then dried under reduced pressure to give the title compound (508 mg) having the following physical property values.
¹H-NMR (DMSO-d6): 11.70, 9.73, 9.65, 7.89 - 7.75, 7.35 - 7.27, 7.00 - 6.96, 6.75, 6.38, 6.00, 5.92, 5.70 - 5.68, 3.32, 3.17 - 2.93.

### [Measurement of physical property data]

With respect to each of the compounds of Examples 1(11), 2, 3, 4 and 5 of which a crystal had been obtained, the physical property data were measured under the following conditions.
(1) Powder X-ray diffraction spectrum:
   Device: SmartLab, manufactured by Rigaku
   Target: Cu
   Voltage: 45 kV
   Current: 200 mA
(2) Differential scanning calorimetry (DSC):
   Device: a differential scanning calorimeter DSC822e, manufactured by METTLER TOLEDO
   Amount of sample: about 1 mg
   Sample cell: aluminum pan 40 µL
   Flow rate of nitrogen gas: 40 ml/min
   Temperature rising rate: 10°C/min
(3) Thermogravimetry (TG):
   Device: a thermogravimeter TGA851e, manufactured by METTLER TOLEDO
   Amount of sample: about 2 mg
   Sample cell: aluminum cell (without lid)
   Flow rate of nitrogen gas: 60 mL/min
   Temperature rising rate: 10°C/min

In the present invention, the crystal form of each of a salt, a solvate and a cocrystal of (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one is specified on the basis of the physical chemical data described in the description. However, it should be noted that each of the spectral data may vary slightly by its nature and therefore should not be interpreted strictly.

For example, with respect to powder X-ray diffraction spectrum data, by its nature, in the determination of crystal identification, a diffraction angle (2θ) or an overall pattern is important and a relative intensity may vary slightly depending on the direction of the growth of a crystal, the size of a grain and the conditions for measurement.

Similarly, with respect to DSC data, in the determination of crystal identification, an overall pattern is important and the data may vary slightly depending on the conditions for measurement.

Therefore, in the compounds of the present invention, those compounds which have similar powder X-ray diffraction spectra or DSC patterns to each other on the whole are included in the compounds of the present invention.

Each of a value for a diffraction angle (2θ (degree)) in a powder X-ray diffraction pattern and a value for a temperature (°C) in a DSC described in the description means that the value includes an error range that is generally acceptable in the data measurement method, and means that the value is approximately that diffraction angle or that temperature. For example, the wording "about" or "substantially the same" used in the expression of a diffraction angle (2θ (degree)) in powder X-ray diffraction means ±0.2 degrees in one aspect and ±0.1 degrees in another aspect. The wording "about" used in the expression of an onset temperature (°C) or an endothermic peak temperature (°C) in a DSC analysis means ±10°C in one aspect, ±5°C in another aspect, and ±2°C in still another aspect.

### Example 1(11): Data of (3S)-3-[2-(6-amino-2-fluoro-3-pyridinyl)-4-fluoro-1H-imidazol-5-yl]-7-[5-chloro-2-(1H-tetrazol-1-yl)-phenyl]-2,3-dihydro-5(1H)-indolizinone dihydrate

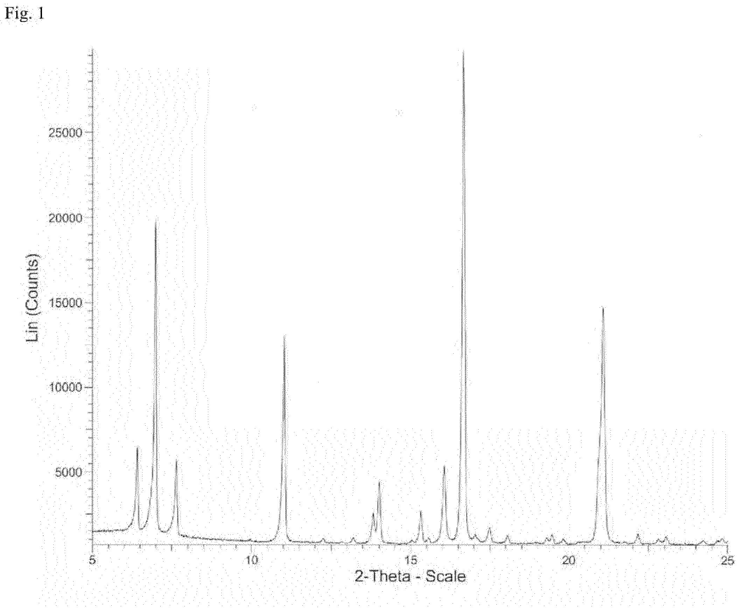

In a powder X-ray diffraction spectrum, this compound showed a powder X-ray diffraction spectrum shown in Fig. 1 and diffraction angles (2θ) and relative intensities shown in Table 2.

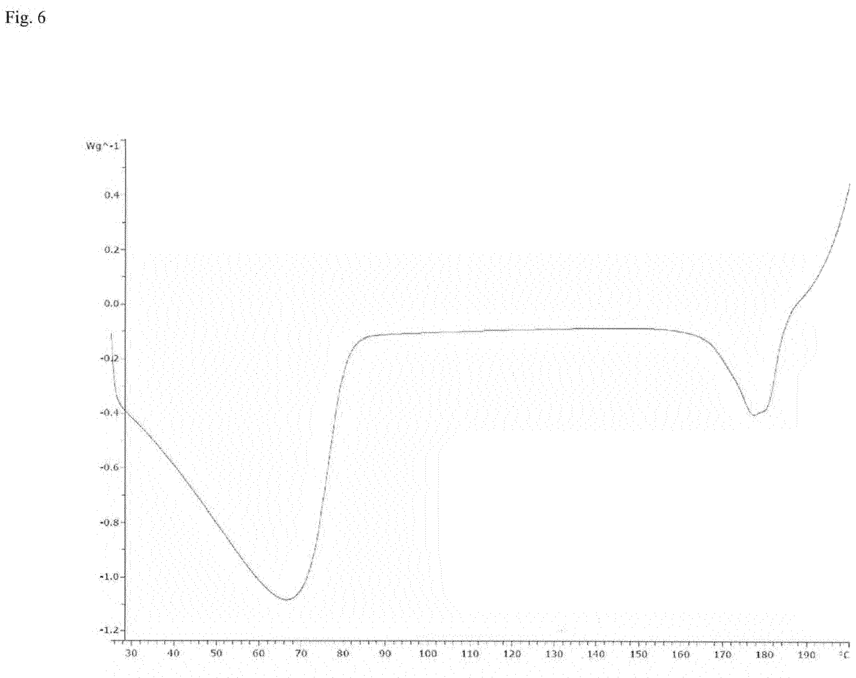

In differential scanning calorimetry, as shown in a chart shown in Fig. 6, this compound showed heat absorption in a temperature range from about room temperature to about 87°C and showed heat absorption and heat generation from about 155°C.

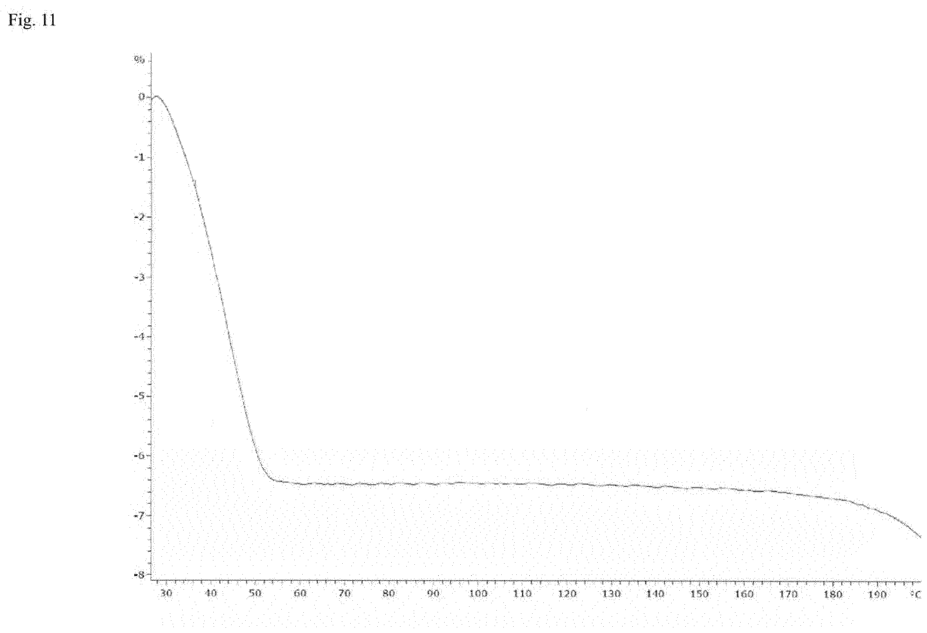

In thermogravimetry, as shown in a chart shown in Fig. 11, this compound showed a decrease in weight by about 6.5% (corresponding to 1.7 molecules of water) from about room temperature to about 60°C.

**[Table 2]**

| Diffraction angle (2θ) (degree) | Relative intensity (%) |
|---|---|
| 6.41 | 21.52 |
| 6.99 | 64.50 |
| 7.65 | 18.91 |
| 11.02 | 40.04 |
| 13.82 | 8.48 |
| 14.00 | 14.21 |
| 15.31 | 8.71 |
| 16.05 | 17.51 |
| 16.67 | 100.00 |
| 21.07 | 49.04 |

### Example 2: Data of (3R)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-α-resorcylic acid (2/1)

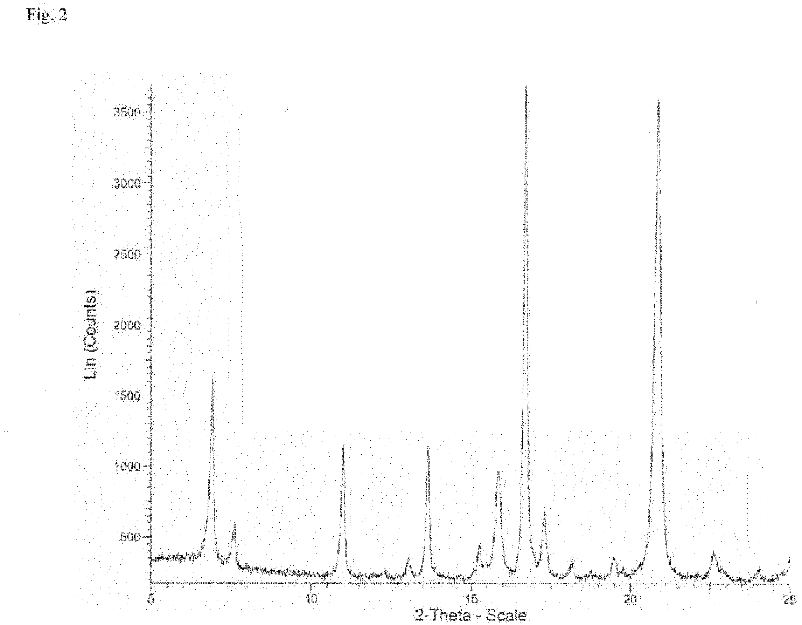

In a powder X-ray diffraction spectrum, this compound showed a powder X-ray diffraction spectrum shown in Fig. 2 and diffraction angles (2θ) relative intensities shown in Table 3.

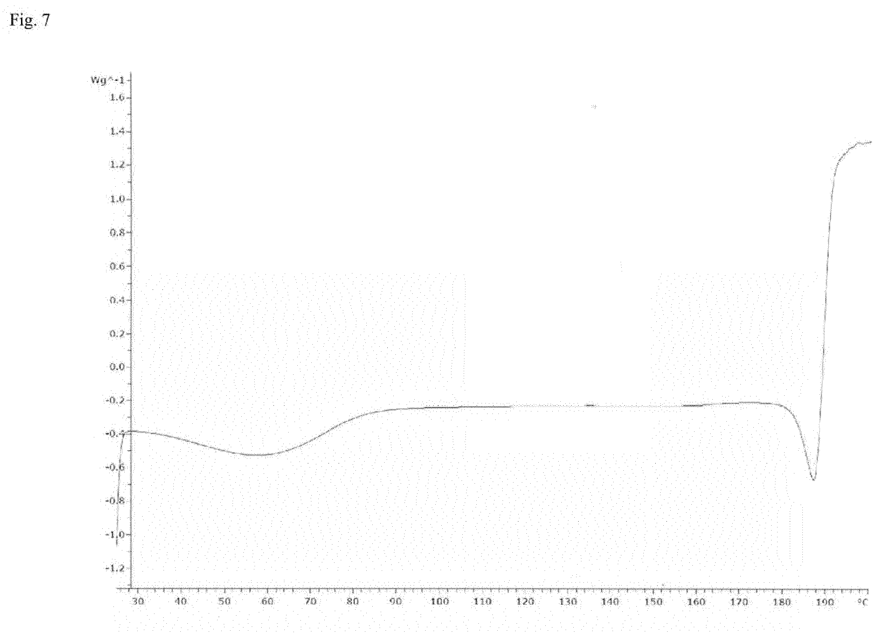

In differential scanning calorimetry, as shown in a chart shown in Fig. 7, this compound showed broad heat absorption in a temperature range from about room temperature to about 90°C, and showed heat absorption and heat generation from about 175°C.

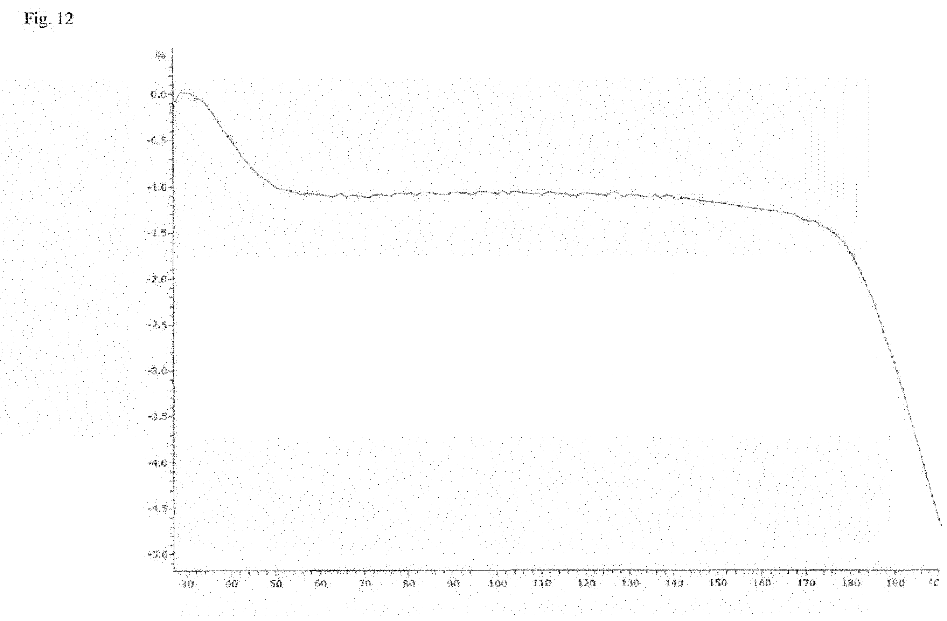

In thermogravimetry, as shown in a chart shown in Fig. 12, this compound showed a decrease in weight by about 1.1% from about room temperature to about 63°C.

**[Table 3]**

| Diffraction angle (2θ) (degree) | Relative intensity (%) |
|---|---|
| 6.90 | 41.47 |
| 7.61 | 16.71 |
| 11.00 | 29.36 |
| 13.64 | 30.03 |
| 15.25 | 11.91 |
| 16.69 | 98.61 |
| 17.30 | 19.12 |
| 20.87 | 100.00 |

### Example 3: Data of (3R)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-gentisic acid (2/1)

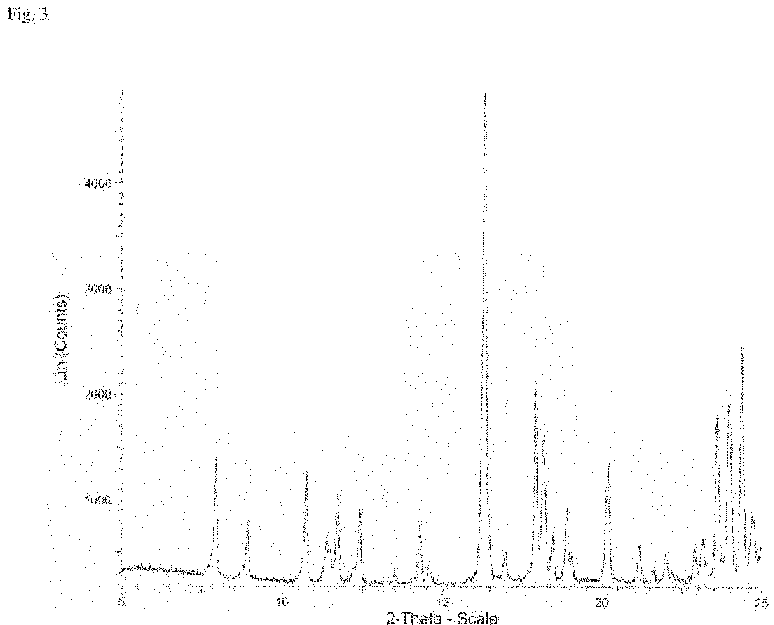

In a powder X-ray diffraction spectrum, this compound showed a powder X-ray diffraction spectrum shown in Fig. 3 and showed diffraction angles (2θ) and relative intensities shown in Table 4.

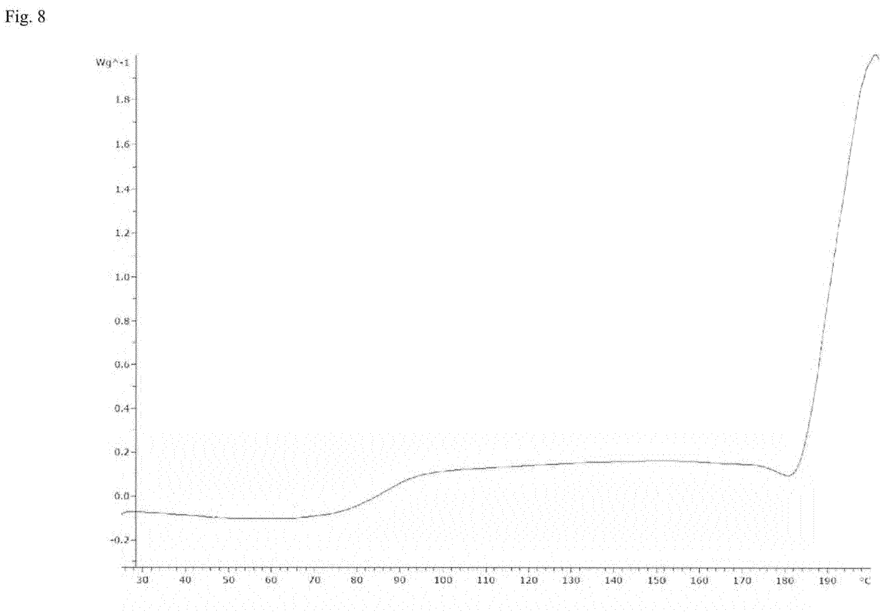

In differential scanning calorimetry, as shown in a chart shown in Fig. 8, this compound showed broad heat absorption in a temperature range from about room temperature to about 100°C and showed heat absorption and heat generation from about 155°C.

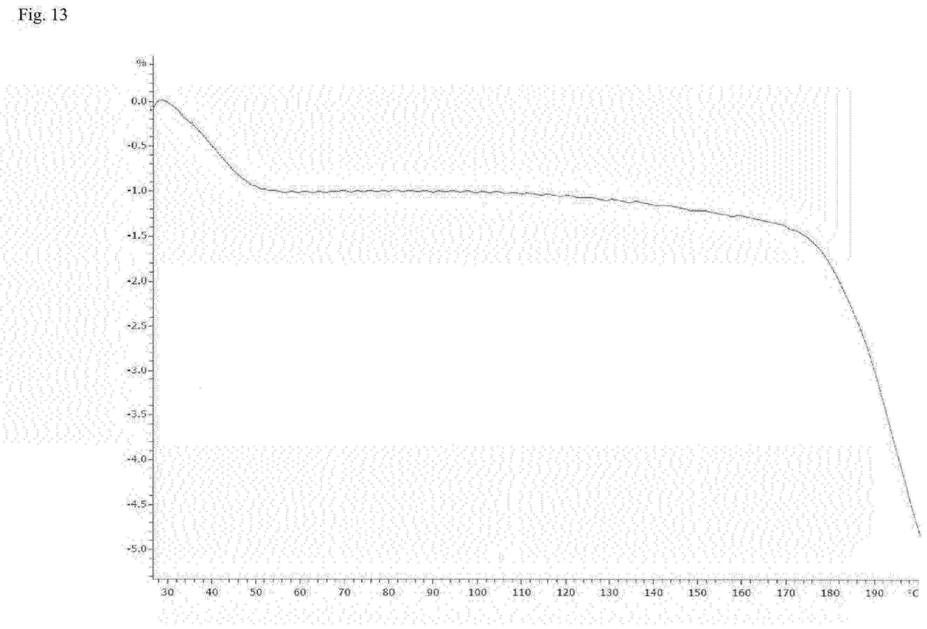

In thermogravimetry, as shown in a chart shown in Fig. 13, this compound showed a decrease in weight by about 1.0% from about room temperature to about 56°C.

**[Table 4]**

| Diffraction angle (2θ) (degree) | Relative intensity (%) |
|---|---|
| 7.94 | 27.55 |
| 8.93 | 16.76 |
| 10.75 | 26.52 |
| 11.73 | 22.60 |
| 12.41 | 17.72 |
| 14.30 | 16.30 |
| 16.32 | 100.00 |
| 16.98 | 11.25 |
| 17.92 | 44.57 |
| 18.92 | 19.71 |
| 20.19 | 28.55 |
| 21.17 | 11.86 |
| 22.00 | 10.83 |
| 23.6 | 38.69 |
| 24.01 | 42.23 |
| 24.37 | 52.03 |
| 24.73 | 18.66 |
| 24.98 | 11.69 |

### Example 4: Data of (3S)-3-[2-(6-amino-2-fluoropyridin-3-yl)-4-fluoro-1H-imidazol-5-yl]-7-[5-chloro-2-(1H-tetrazol-1-yl)-phenyl]-2,3-dihydroindolizin-5(1H)-one-nicotinamide (2/1)

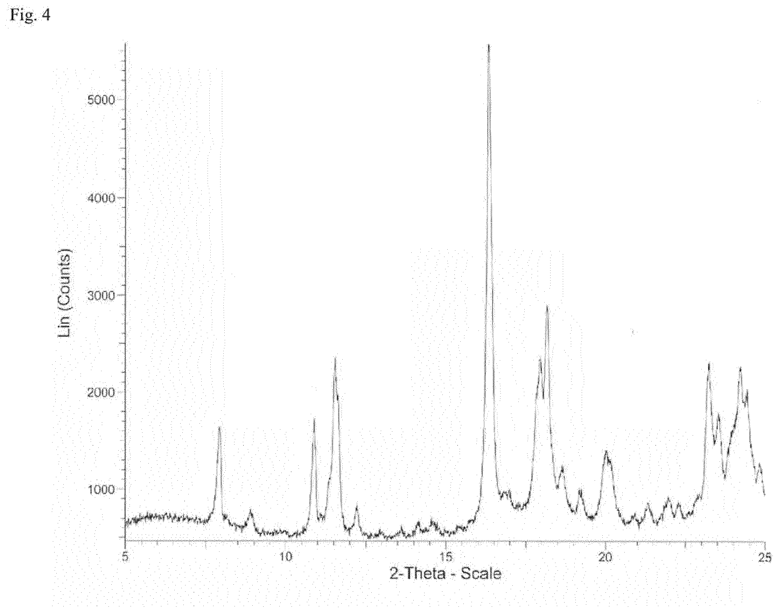

In powder X-ray diffraction spectrum, this compound showed a powder X-ray diffraction spectrum shown in Fig. 4 and showed diffraction angles (2θ) and relative intensities shown in Table 5.

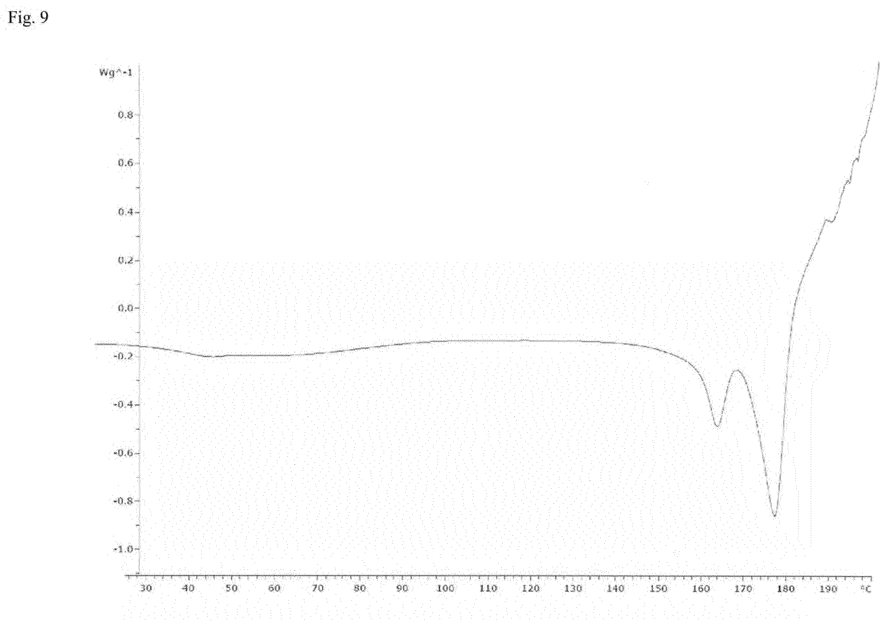

In differential scanning calorimetry, as shown in a chart shown in Fig. 9, this compound showed heat absorption and heat generation from about 144°C.

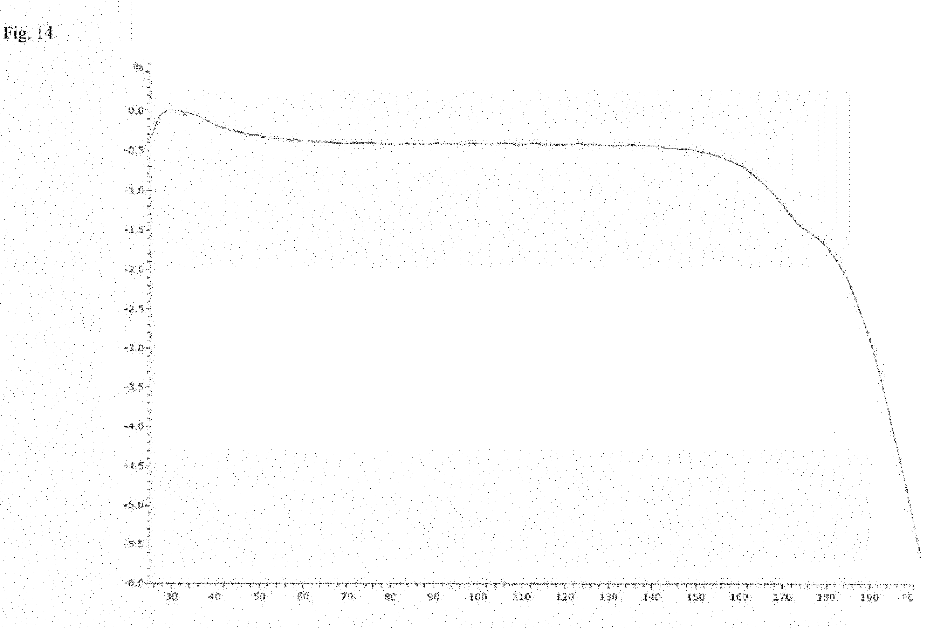

In thermogravimetry, as shown in a chart shown in Fig. 14, this compound showed a decrease in weight by about 0.4% (i.e., less than 1%) from about room temperature to about 65°C.

**[Table 5]**

| Diffraction angle (2θ) (degree) | Relative intensity (%) |
|---|---|
| 7.92 | 29.11 |
| 10.89 | 30.42 |
| 11.55 | 42.24 |
| 16.32 | 100.00 |
| 17.96 | 42.65 |
| 18.17 | 52.11 |
| 18.64 | 21.47 |
| 20.03 | 25.35 |
| 23.25 | 41.52 |
| 23.53 | 32.14 |
| 24.23 | 40.18 |

### Example 5: Data of (3S)-3-[2-(6-amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-3-hydroxybenzoic acid 1/1

In powder X-ray diffraction spectrum, this compound showed a powder X-ray diffraction spectrum shown in Fig. 5 and showed diffraction angles (2θ) and relative intensities shown in Table 6.

In differential scanning calorimetry, as shown in a chart shown in Fig. 10, this compound showed heat generation in a temperature range from about 200°C to about 234°C. The onset temperature was 213.6°C, and the exothermic peak temperature was 214.7°C.

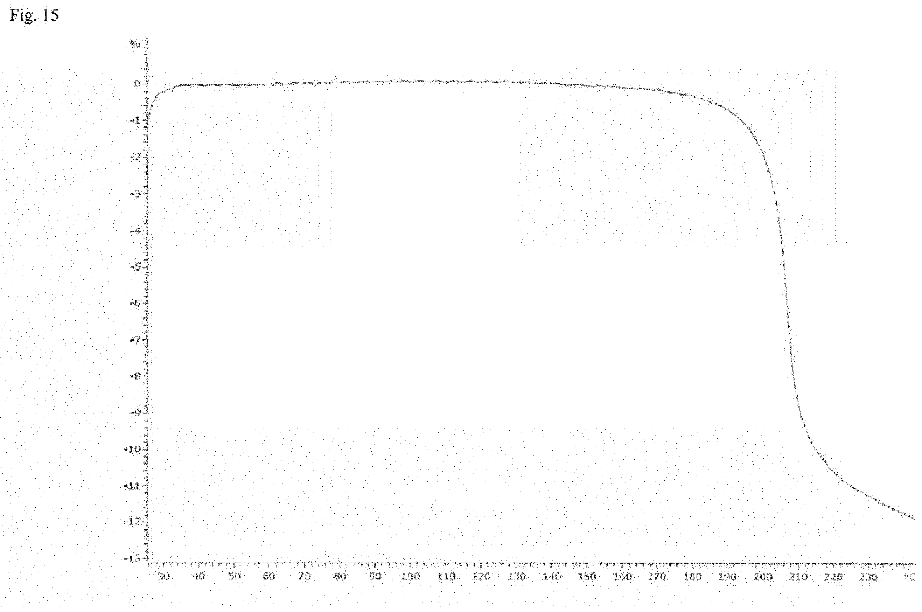

In thermogravimetry, as shown in a chart shown in Fig. 15, a decrease in weight was not observed from about room temperature to about 156°C.

**[Table 6]**

| Diffraction angle (2θ) (degree) | Relative intensity (%) |
|---|---|
| 9.39 | 86.80 |
| 13.28 | 14.36 |
| 14.09 | 63.82 |
| 14.67 | 17.39 |
| 15.11 | 56.21 |
| 15.77 | 38.46 |
| 16.46 | 100.00 |
| 17.37 | 31.65 |
| 17.69 | 41.87 |
| 18.01 | 31.87 |
| 18.39 | 52.02 |
| 18.87 | 34.81 |
| 19.28 | 24.39 |
| 19.78 | 16.19 |
| 20.62 | 47.91 |
| 20.85 | 47.23 |
| 22.41 | 37.21 |
| 22.80 | 18.95 |
| 23.16 | 22.64 |
| 24.17 | 67.06 |
| 24.96 | 13.75 |
| 25.53 | 29.97 |
| 25.76 | 65.76 |
| 26.28 | 27.71 |
| 27.23 | 85.81 |
| 27.57 | 18.56 |
| 27.85 | 23.52 |
| 28.62 | 12.27 |
| 29.50 | 24.77 |
| 31.02 | 21.21 |

The single crystal X-ray structural analysis data of the compound of Example 5 are shown in Tables 7 to 10 below.

### [Measurement conditions]

Device: a single crystal X-ray analysis device SuperNova, manufactured by Rigaku
Target: Cukα (λ = 1.54184Å)
Measurement temperature: 100 K
Analysis software: Olex2[1]
1.Dolomanov, O.V., Bourhis, L.J., Gildea, R.J, Haward, J.A.K. & Puschmann, H. (2009), J. Appl. Cryst. 42, 339-341.

Crystallographic data are as follows.

**[Table 7]**

| Unit lattice data | |
|---|---|
| T(K) | 100 |
| a (Å) | 6.86859 (17) |
| b (Å) | 18.6585 (4) |
| c (Å) | 22.3480 (6) |
| α° | 90 |
| β° | 90 |
| γ° | 90 |
| Vm | 716 |
| Z' | 1 |
| SG | P212121 |
| Dcalc | 1.498 |
| R | 0.0419 |

**[Table 8]**

| Positional parameter 1 | | | |
|---|---|---|---|
| Atom | x | y | z |
| C101 | 2097 | 8747.8 | -348.4 |
| F2 | 4091 | 7155.4 | 5213.7 |
| F1 | 2140 | 5342.5 | 3056.9 |
| O1 | 3881 | 7577.7 | 2882.2 |
| O2 | 13339 | 9443.9 | 1199.6 |
| O4 | 7156 | 8278 | 2784.4 |
| O3 | 7023 | 8350 | 1783.8 |
| N6 | 2976 | 6663.8 | 4105.7 |
| N8 | 6966 | 6840.5 | 5593 |
| N5 | 1059 | 6996.1 | 2662.8 |
| N7 | 4089 | 5587.1 | 3884.7 |
| N1 | 2157 | 5813.4 | 651.8 |
| N9 | 9916 | 6593.9 | 6011.1 |
| C18 | 4278 | 6151.9 | 4252.6 |
| N2 | 3288 | 5631.5 | 1130.8 |
| C16 | 1931 | 6440.9 | 3615.1 |
| C4 | 2170 | 7800.1 | 557.5 |
| C3 | 2096 | 7113.8 | 805.3 |
| C20 | 5659 | 6720.2 | 5179.5 |
| N3 | 3010 | 4955.9 | 1218.1 |
| C27 | 10668 | 9017 | 2832.4 |
| C9 | 3071 | 7363.7 | 1855.9 |
| C17 | 2665 | 5781.2 | 3504.3 |

**[Table 9]**

| Positional parameter 2 | | | |
|---|---|---|---|
| Atom | x | y | z |
| C8 | 1791 | 7045.4 | 1467.9 |
| N4 | 1726 | 4679.3 | 812 |
| C19 | 5742 | 6220.5 | 4719.9 |
| C11 | -240 | 6677.3 | 2276.9 |
| C10 | 2747 | 7331.4 | 2488.1 |
| C5 | 2163 | 7882.5 | -56.6 |
| C26 | 9777 | 8840.3 | 2288.9 |
| C15 | 483 | 6911.3 | 3302.1 |
| C30 | 7853 | 8474.5 | 2254.2 |
| C21 | 8578 | 6431.3 | 5594.8 |
| C12 | 104 | 6690.5 | 1674.6 |
| C29 | 13412 | 9491.3 | 2286.2 |
| C23 | 7427 | 5792 | 4734.7 |
| C28 | 12476 | 9342.5 | 2824.3 |
| C6 | 2156 | 7299 | -444.1 |
| C24 | 12510 | 9319.3 | 1745.4 |
| C7 | 2171 | 6615 | -201.5 |
| C25 | 10687 | 9007.2 | 1749.1 |
| C13 | -1866 | 6344 | 2620.1 |
| C2 | 2126 | 6526.8 | 416.8 |
| C14 | -1605 | 6617 | 3260.6 |
| C22 | 8837 | 5885.6 | 5162 |
| C1 | 1239 | 5224 | 475 |

**[Table 10]**

| Positional parameter 3 | | | |
|---|---|---|---|
| Atom | x | y | z |
| H2 | 14187 | 9751 | 1233 |
| H4A | 6115 | 8069 | 2739 |
| H6 | 2825 | 7065 | 4289 |
| H9A | 9713 | 6942 | 6256 |
| H9B | 10977 | 6350 | 6033 |
| H4 | 2223 | 8200 | 805 |
| H27 | 10053 | 8917 | 3194 |
| H9 | 4157 | 7602 | 1708 |
| H15 | 459 | 7383 | 3495 |
| H12 | -750 | 6472 | 1408 |
| H29 | 14635 | 9705 | 2287 |
| H23 | 7588 | 5436 | 4447 |
| H28 | 13075 | 9464 | 3184 |
| H6A | 2141 | 7366 | -856 |
| H7 | 2211 | 6216 | -451 |
| H25 | 10062 | 8908 | 1389 |
| H13A | -1781 | 5825 | 2606 |
| H13B | -3116 | 6490 | 2460 |
| H14A | -1790 | 6229 | 3545 |
| H14B | -2543 | 6991 | 3347 |
| H22 | 9940 | 5597 | 5167 |
| H1 | 370 | 5202 | 156 |

In general, for demonstrating that two components exist in the form of a cocrystal, it is required to show a requirement represented by the following formula are satisfied: a/b > 1.08 (Mol Pharm. 2007 May-Jun; 4(3):317-22) and/or ΔDc-o > 0.08Å (Mol Pharm. 2007 May-Jun; 4(3):323-38).

The values of a/b and ΔDc-o determined from the above-shown atomic coordinate tables are a/b ≈ 1.091 and ΔDc-o ≈ 0.208. Therefore, it is obvious that the compound of Example 1(10) and 3-hydroxybenzoic acid exist as a cocrystal.

Comparison is made between powder X-ray diffraction spectrum data converted from the single crystal X-ray structural analysis data and actually measured powder X-ray diffraction spectrum data of the compound of Example 5, and it is found that scattering peaks of these data match each other. Therefore, it is demonstrated that the compound of Example 5 is a cocrystal.

Furthermore, from the measurement results of solid ¹H-NMR and ¹³C-NMR (CP/MAS) (CP: cross-polarization, MAS: magic-angle spinning) and ¹H-¹³C CP-HETCOR (HETCOR: Heteronuclear Correlation Spectroscopy), it is also confirmed that the compound of Example 1(10) and 3-hydroxybenzoic acid exist as a cocrystal.

### Physical Chemical Example 1: Evaluation of hygroscopicity

An isothermal absorption curve measurement (DVS) was carried out under the following conditions.

### (Conditions)

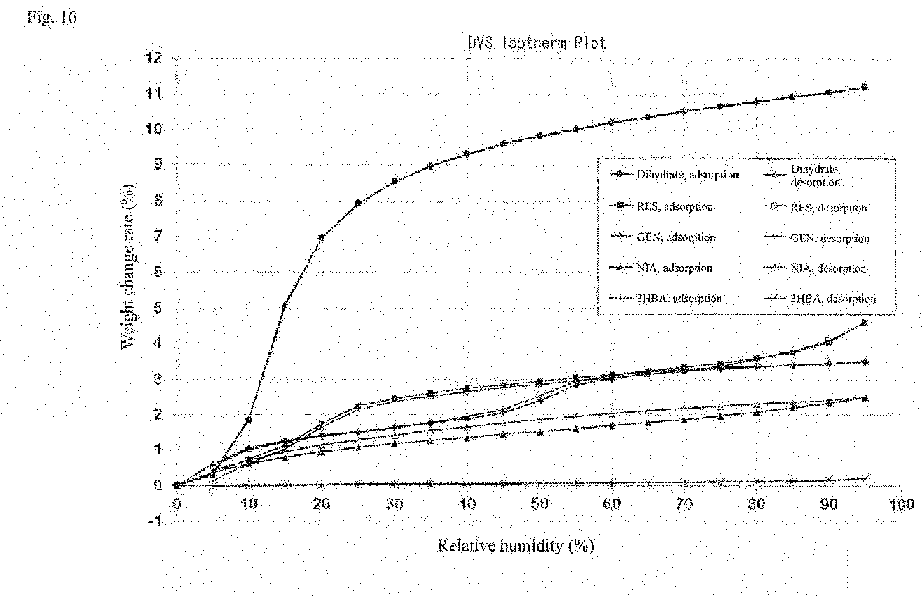

Device: DVS Intrinsic, manufactured by SMS
Measurement temperature: 25°C
Measurement range: relative humidity of 0 to 95%
Interval of measurement: relative humidity of 5%
As the result of this evaluation, as shown in Fig. 16, the weight change rate of the compound of Example 5 in this evaluation was less than 1% and it was found that only a cocrystal of 3HBA had low hygroscopicity.

### Physical Chemical Example 2: Evaluation of solubility

The measurement of solubility was carried out under the following conditions.

About 3 mg of each of the compounds of the present invention was weighed in a test tube, then a stirrer and 5 mL of each of an artificial intestinal juice (FaSSIF; Reference Document 1: Pharmaceutical Research, vol. 20, pp. 1674-1680, 2003; Reference Document 2: Biological & Pharmaceutical Bulletin, vol. 34, pp. 401-407, 2011) and JP 1st fluid which had been warmed to 37°C were added to the test tube, and then the test tube was sealed hermetically. The test tube was installed in a solubility test device (Gilson, Quad-Z 215), and the solution was stirred at 37°C and 700 rpm. After 0.25, 0.5, 1, 3, 6 and 24 hours, an aliquot of the solution in the test tube was collected. The collected solution was filtrated through a filter, and a filtrate was diluted two-folds with acetonitrile. A diluted solution of the artificial intestinal juice was centrifuged at 3000 rpm for 5 minutes, and a supernatant was used as a sample solution. A diluted solution of the JP 1st fluid was used as is as a sample solution. Solubility was calculated by a high-performance liquid chromatography method.

### [Measurement conditions]

Device: Shimadzu HPLC NexeraXRseries
Column: Waters Xbridge ShieldRP18 (4.6 mm (inner diameter) × 50 mm, 3.5 µm)
Column temperature: 40°C
Component of mobile phase: 20 mM potassium dihydrogen phosphate (pH 3.0)/acetonitrile (0 minute: 70/30, 5 minutes: 25/75)
UV: 285 nm
Flow rate: 1.0 mL/min
Sample rack temperature: 15°C
Sample injection amount: 10 µL
Measurement time: 5 minutes
Retention time: 2.6 minutes

The solubilities of the compounds of Example 1(11), Example 4 and Example 5 after 24 hours are shown in Table 11 below.

**[Table 11]**

| Compound | JP 1st fluid (µg/m) | Artificial intestinal juice (µg/m) |
|---|---|---|
| Example 1(11) | 41 | 47 |
| Example 4 | 77 | 60 |
| Example 5 | 75 | 60 |

The solubility of the compound of Example 1(11) in the JP 1st fluid was 41 µg/mL, and the solubilities of the compounds of Example 4 and Example 5 in the JP 1st fluid were 77 µg/mL and 75 µg/mL, respectively. The solubility of the compound of Example 1(11) in the artificial intestinal juice was 47 µg/mL, and the solubility of each of the cocrystals of the compounds of Example 4 and Example 5 in the artificial intestinal juice was 60 µg/mL.

### Physical Chemical Example 3: Evaluation of oral absorption

The measurement of oral absorption was carried out under the following conditions.

### [Methods for preparation/administration and blood collection]

Suspensions of the compounds of Example 1(11), Example 4 and Example 5 were prepared. Each of the suspensions was prepared with a 0.5% aqueous methyl cellulose solution at a concentration of 0.33 mg/mL (in terms of free base content). Each of the suspensions was administered to monkeys (cynomolgus monkeys/male), which had been fasted since the day before, in the stomach thereof forcibly with a sonde. The amount of administration was adjusted depending on the body weight of each of the monkeys so that the dosage amount became 1 mg/kg.

After the administration of the suspensions (liquid pharmaceuticals), at the timing of 0.25, 0.5, 1, 2, 4, 7 and 24 hours after the administration, blood (0.5 mL) was collected from the cephalic vein with a heparin-added syringe. The collected blood was centrifuged at 10000 G for 5 minutes to separate plasma from the blood. In the evaluation of the oral absorption of the test compounds, data obtained when the test compounds were administered intravenously at the same dose amounts (1 mg/kg) were employed. The oral absorption rate (%) was calculated by dividing an AUC (area under the blood concentration-time curve) value obtained in the oral administration by an AUC value obtained in the intravenous administration and then multiplying the resultant value by 100.

### [Preparation/analysis of analysis samples]

Water/acetonitrile (19/1) (200 µL) and acetonitrile containing an internal standard substance (a structural analogue of the compound of Example 1(10)) (10 µL) were added to plasma (10 µL), and the resultant solution was stirred. The whole volume of the solution was added to Oasis HLB µElution Plate through which acetonitrile (200 µL) and purified water (200 µL) had been passed, and was then sucked under reduced pressure. Purified water (200 µL) was added to the solution, and the resultant solution was washed by sucking under reduced pressure. Acetonitrile (200 µL) was added to the solution, the resultant solution was sucked under reduced pressure, and an eluate was collected in a 96-well plate. The eluate (10 µL) was added to a 0.1% aqueous formic acid solution/acetonitrile (2/1) solution (290 µL), and the resultant solution was stirred and was then analyzed by LC/MS/MS.

The analysis by LC/MS/MS was carried out under the following conditions.

### [Conditions for LC/MS/MS]

Measurement device: API-5000 (manufactured by Applied Biosystems/MDS SCIEX) Analysis column: Cadenza CD-C18 (2.0 mm I.D. × 100 mm, 3 µm)
Flow rate: 0.4 mL/min.
Components of mobile phase: a 0.1% aqueous formic acid solution/acetonitrile (0 minute: 65/35, 4.0 minutes: 65/35, 4.1 minutes: 10/90, 5.0 minutes: 10/90, 5.1 minutes: 65/35, 7.0 minutes: 65/35)
Scan type: MRM (Multiple Reaction Monitoring)
Polarity: Positive

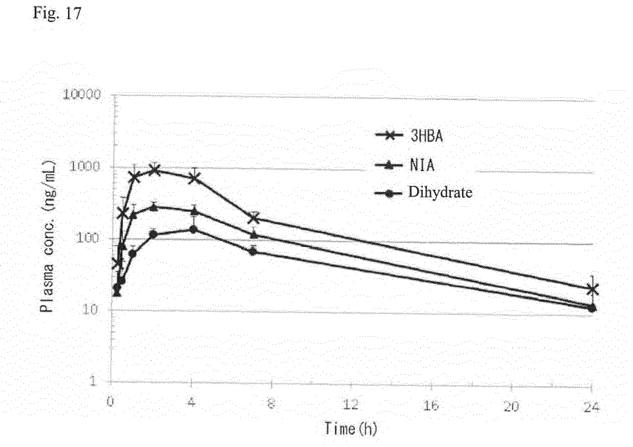

The results of this evaluation are shown in Fig. 17. The oral absorption rates (%) of the compounds of Example 1(11) and Example 4, among the compounds of Example 1(11), Example 4 and Example 5, were 26% and 44%, respectively. In contrast, the oral absorption rate (%) of the compound of Example 5 was almost 100%.

### [Preparation Examples]

### Preparation Example 1:

### Tablets containing 5 mg of compound A

The below-mentioned components were mixed together and were then compressed into tablets by the conventional method. In this manner, 10,000 tablets containing the active ingredient in an amount of 5 mg per tablet were produced.
- (3S)-3-[2-(6-Amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-3-hydroxybenzoic acid (1/1): 50 g
- Carboxymethylcellulose calcium (disintegrating agent): 20 g
- Magnesium stearate (lubricant): 10 g
- Microcrystalline cellulose: 920 g

### Preparation Example 2:

Injection containing 20 mg of compound A

The below-mentioned components were mixed together by the conventional method, and then the resultant solution was sterilized by the conventional method, was then packed in ampules at a volume of 5 mL per ampule, and was then freeze-dried by the conventional method. In this manner, 10,000 ampules each containing the active ingredient in an amount of 20 mg per ampule were produced.
- (3 S)-3 - [2-(6-Amino-2-fluoropyridin-3-yl)-4-fluoro-1H-imidazol-5-yl]-7-[5-chloro-2-(1H-tetrazol-1-yl)phenyl]-2,3-dihydroindolizin-5(1H)-one-3-hydroxybenzoic acid (1/1): 200 g
- Mannitol: 20 g
- Distilled water: 50 L

### INDUSTRIAL APPLICABILITY

The compound A has an extremely potent inhibitory activity against blood coagulation factor XIa, and is also superior in low hygroscopicity and oral absorption. Therefore, the compound A is very useful as an active ingredient for a pharmaceutical.

## Claims

1. (3S)-3-[2-(6-Amino-2-fluoropyridin-3-yl)-4-fluoro-1H-imidazol-5-yl]-7-[5-chloro-2-(1H-tetrazol-1 -yl)phenyl] -2,3 -dihydroindolizin-5(1H)-one-3-hydroxybenzoic acid (1/1).

2. The compound according to claim 1, wherein the compound is in a crystal form.

3. The compound according to claim 2, wherein the crystal form has at least two diffraction peaks at diffraction angles (2θ) of about 9.4, about 14.1, about 15.8, about 16.5, about 17.4, about 18.4, about 20.6 and about 22.4 degrees in a powder X-ray diffraction spectrum.

4. The compound according to claim 2, wherein the crystal form has diffraction peaks at diffraction angles (2θ) of about 9.4, about 13.3, about 14.1, about 14.7, about 15.1, about 15.8, about 16.5, about 17.4, about 17.7, about 18.0, about 18.4, about 18.9, about 19.3, about 19.8, about 20.6, about 20.9, about 22.4, about 22.8, about 23.2, about 24.2, about 25.0, about 25.5, about 25.8, about 26.3, about 27.2, about 27.6, about 27.9, about 28.6, about 29.5 and about 31.0 degrees in a powder X-ray diffraction spectrum.

5. The compound according to claim 2, wherein the crystal form is **characterized by** having a powder X-ray diffraction spectrum chart shown in Fig. 5.

6. The compound according to claim 2 or 3, wherein the crystal form has an onset temperature of about 214°C or an exothermic peak temperature of about 215°C in differential scanning calorimetry.

7. The compound according to claim 6, wherein the crystal form is **characterized by** having a differential scanning calorimetry chart shown in Fig. 10.

8. The compound according to claims 1 to 7, wherein the compound is a cocrystal.

9. A pharmaceutical composition comprising the compound according to any one of claims 1 to 8.

10. The pharmaceutical composition according to claim 9, wherein the pharmaceutical composition is a blood coagulation factor XIa inhibitor.

11. The pharmaceutical composition according to claim 10, wherein the pharmaceutical composition is a prophylactic and/or therapeutic agent for a blood coagulation factor XIa-related disease.

12. The pharmaceutical composition according to claim 11, wherein the blood coagulation factor XIa-related disease is a thromboembolic disease.

13. The pharmaceutical composition according to claim 12, wherein the thromboembolic disease is arterial cardiovascular thromboembolic disorder, venous cardiovascular thromboembolic disorder, arterial cerebrovascular thromboembolic disorder, venous cerebrovascular thromboembolic disorder or thromboembolic disorder in the heart chamber or in the peripheral circulation.

14. The pharmaceutical composition according to claim 12, wherein the thromboembolic disease is cerebral stroke, cerebral thrombosis, cerebral embolism, venous thrombosis, venous thromboembolism, deep venous thrombosis, or a thrombosis and/or a thromboembolism induced by a treatment in which blood is exposed on a surface of an artificial object.
